# EUROPEAN PATENT APPLICATION

(11) **EP 1 284 289 A1**
(43) Date of publication of application: **19.02.2003**
(21) Application number: 01271441.6
(22) Date of filing: 21.12.2001
(51) Int. Cl.: C12N 15/11, C07K 16/18

(54) **METHOD OF EXAMINING ALLERGIC DISEASE**

(30) Priority: 21.12.2000 JP 2000389476
(71) Applicant: Genox Research, Inc., Ibaraki 300-2635 (JP); Japan as represented by General Director of Agency of National Center for Child Health and Development, Tokyo 157-8535 (JP)
(72) Inventor: MATSUMOTO, Yoshiko, c/o Genox Research Inc. Teikyo, Kawasaki-shi, Kanagawa 216-0001 (JP); IMAI, Yukiho, c/o Genox Research Inc. Teikyo, Kawasaki-shi, Kanagawa 216-0001 (JP); OSHIDA, Tadahiro, c/o Genox Research Inc. Teikyo, Kawasaki-shi, Kanagawa 216-0001 (JP); SUGITA, Yuji, c/o Genox Research Inc., Kawasaki-shi, Kanagawa 216-0001 (JP); NAGASU, Takeshi, c/o Eisai Co. Ltd., Tsukuba-shi, Ibaraki 300-2635 (JP); TSUJIMOTO, Gozoh, Nat.Ctr for Child Health and Dev, Tokyo 154-8509 (JP)
(74) Representative: Warcoin, Jacques
(86) International application number: JP0111286
(87) International publication number: WO02050269

(57) **Abstract**

The present inventors collected blood samples from a plurality of normal healthy subjects and allergic disease patients, and conducted the differential display analysis to search for a gene that shows a difference in its expression among them. As a result, the inventors succeeded in isolating the B1153 gene whose expression level is significantly high in the allergic disease patient group. The inventors found it possible to utilize this gene in testing for an allergic disease, and screening for a candidate compound for a therapeutic agent for an allergic disease.

SEQUENCE LISTING

<110> Genox Research, Inc.
Japan as Represented by General Director of National Children's
Hospital

<120> METHOD OF TESTING FOR ALLERGIC DISEASE

<130> G1-A0007P

<140>
<141>

<150> JP 2000-389476
<151> 2000-12-21

<160> 26

<170> PatentIn Ver. 2.1

<210> 1
<211> 3596
<212> DNA
<213> Homo sapiens

<220>
<221> CDS

<222> (86)..(2980)

<400> 1

cttcctagtg tcagggccag ctgtgtagtg gctcggtgtg atttgttagc tctttgaggc 60

agggtaccct cctcaggatt tcgat atg caa aaa atc aaa tct ctc atg acc 112
Met Gln Lys Ile Lys Ser Leu Met Thr
1               5

cga cag ggt ctg aaa agc cct caa gaa agc ctc agt gat ctt ggt gcc 160
Arg Gln Gly Leu Lys Ser Pro Gln Glu Ser Leu Ser Asp Leu Gly Ala
10                  15                  20                  25

ata gag agt ctc cgg gtc cct gga aag gaa gaa ttc agg gaa ctt cga 208
Ile Glu Ser Leu Arg Val Pro Gly Lys Glu Glu Phe Arg Glu Leu Arg
                30                  35                  40

gaa cag cca agt gac cct caa gct gaa caa gag ctt att aat agt att 256
Glu Gln Pro Ser Asp Pro Gln Ala Glu Gln Glu Leu Ile Asn Ser Ile
            45                  50                  55

gaa caa gta tat ttt tct gtg gat tca ttt gat att gtt aaa tat gag 304
Glu Gln Val Tyr Phe Ser Val Asp Ser Phe Asp Ile Val Lys Tyr Glu
        60                  65                  70

ctg gag aag ctt cca cct gtt ctc aat ttg caa gaa tta gag gcg tat 352

Leu Glu Lys Leu Pro Pro Val Leu Asn Leu Gln Glu Leu Glu Ala Tyr
    75                  80                  85

aga gac aaa ttg aaa caa cag caa gct gca gta tct aaa aaa gtg gca 400
Arg Asp Lys Leu Lys Gln Gln Gln Ala Ala Val Ser Lys Lys Val Ala
90                  95                  100                 105

gat tta atc ctt gaa aaa cag cct gct tat gta aag gaa ctt gaa aga 448
Asp Leu Ile Leu Glu Lys Gln Pro Ala Tyr Val Lys Glu Leu Glu Arg
                110                 115                 120

gtt acc tca ttg cag aca ggt ctt caa tta gct gct gtt atc tgt aca 496
Val Thr Ser Leu Gln Thr Gly Leu Gln Leu Ala Ala Val Ile Cys Thr
            125                 130                 135

aat ggg aga aga cac ttg aat att gca aag gaa ggt ttt act caa gct 544
Asn Gly Arg Arg His Leu Asn Ile Ala Lys Glu Gly Phe Thr Gln Ala
        140                 145                 150

agt tta ggc ctt ctt gca aat caa agg aaa cgt cag ttg ctg att gga 592
Ser Leu Gly Leu Leu Ala Asn Gln Arg Lys Arg Gln Leu Leu Ile Gly
    155                 160                 165

ctt ctg aaa tct ctg aga act ata aaa aca ttg caa aga aca gat gta 640
Leu Leu Lys Ser Leu Arg Thr Ile Lys Thr Leu Gln Arg Thr Asp Val
170                 175                 180                 185

cgg tta agt gaa atg ctg gag gag gaa gat tat cca gga gct att cag 688
Arg Leu Ser Glu Met Leu Glu Glu Glu Asp Tyr Pro Gly Ala Ile Gln
                190                 195                 200

ttg tgc ctt gaa tgt caa aaa gct gcc agc act ttt aaa cat tac agt 736
Leu Cys Leu Glu Cys Gln Lys Ala Ala Ser Thr Phe Lys His Tyr Ser
            205                 210                 215

tgt ata agt gaa ctg aat tca aag ctg caa gat act ttg gaa cag att 784
Cys Ile Ser Glu Leu Asn Ser Lys Leu Gln Asp Thr Leu Glu Gln Ile
        220                 225                 230

gag gaa cag ctg gac gta gct ctt tcc aaa atc tgc aag aat ttt gac 832
Glu Glu Gln Leu Asp Val Ala Leu Ser Lys Ile Cys Lys Asn Phe Asp
    235                 240                 245

att aac cat tat acc aag gtt caa caa gct tat cga ctt ctt gga aaa 880
Ile Asn His Tyr Thr Lys Val Gln Gln Ala Tyr Arg Leu Leu Gly Lys
250                 255                 260                 265

aca cag aca gca atg gat caa ctt cat atg cac ttc acc caa gcc att 928
Thr Gln Thr Ala Met Asp Gln Leu His Met His Phe Thr Gln Ala Ile
                270                 275                 280

cac aac acc gtg ttt caa gtt gtt ctt ggt tat gtg gaa cta tgt gca 976

His Asn Thr Val Phe Gln Val Val Leu Gly Tyr Val Glu Leu Cys Ala
            285                 290                 295

gga aac aca gac aca aaa ttc caa aag ctg caa tat aag gat ctc tgt 1024
Gly Asn Thr Asp Thr Lys Phe Gln Lys Leu Gln Tyr Lys Asp Leu Cys
        300                 305                 310

aca cat gtt aca cca gac agc tat att cca tgc ctt gca gac ctg tgc 1072
Thr His Val Thr Pro Asp Ser Tyr Ile Pro Cys Leu Ala Asp Leu Cys
    315                 320                 325

aaa gca cta tgg gaa gtt atg ctc agc tat tat agg act atg gaa tgg 1120
Lys Ala Leu Trp Glu Val Met Leu Ser Tyr Tyr Arg Thr Met Glu Trp
330                 335                 340                 345

cat gaa aag cat gac aat gag gat act gct tca gct tct gaa ggg agt 1168
His Glu Lys His Asp Asn Glu Asp Thr Ala Ser Ala Ser Glu Gly Ser
                350                 355                 360

aat atg ata ggt act gaa gaa act aat ttt gat cgt ggc tac ata aaa 1216
Asn Met Ile Gly Thr Glu Glu Thr Asn Phe Asp Arg Gly Tyr Ile Lys
            365                 370                 375

aag aaa tta gaa cat gga ctt aca cga ata tgg cag gat gtt cag cta 1264
Lys Lys Leu Glu His Gly Leu Thr Arg Ile Trp Gln Asp Val Gln Leu
        380                 385                 390

aaa gta aaa acc tac ttg ctt gga act gat ttg tct ata ttc aaa tat 1312
Lys Val Lys Thr Tyr Leu Leu Gly Thr Asp Leu Ser Ile Phe Lys Tyr
    395                 400                 405

gat gat ttc atc ttt gtt ttg gat ata atc agc agg ttg atg caa gtt 1360
Asp Asp Phe Ile Phe Val Leu Asp Ile Ile Ser Arg Leu Met Gln Val
410                 415                 420                 425

gga gaa gaa ttt tgt ggt agc aag tct gaa gtt tta cag gaa tct att 1408
Gly Glu Glu Phe Cys Gly Ser Lys Ser Glu Val Leu Gln Glu Ser Ile
                430                 435                 440

aga aaa caa agt gtc aat tat ttc aag aat tac cat aga aca cgg ctc 1456
Arg Lys Gln Ser Val Asn Tyr Phe Lys Asn Tyr His Arg Thr Arg Leu
            445                 450                 455

gat gaa ctg aga atg ttc tta gag aat gag act tgg gaa ctt tgt cct 1504
Asp Glu Leu Arg Met Phe Leu Glu Asn Glu Thr Trp Glu Leu Cys Pro
        460                 465                 470

gtt aag tca aat ttc agc atc ttg caa ctt cat gaa ttt aaa ttc atg 1552
Val Lys Ser Asn Phe Ser Ile Leu Gln Leu His Glu Phe Lys Phe Met
    475                 480                 485

gaa cag tct cgc tcc cca tca gtt tca cct agt aaa cag cca gtc tca 1600

Glu Gln Ser Arg Ser Pro Ser Val Ser Pro Ser Lys Gln Pro Val Ser
490                 495                 500                 505

act tct tca aaa aca gtg acc ttg ttt gag cag tac tgt agt ggt ggg    1648
Thr Ser Ser Lys Thr Val Thr Leu Phe Glu Gln Tyr Cys Ser Gly Gly
                510                 515                 520

aat cca ttt gaa att cag gcc aac cac aaa gat gaa gaa aca gaa gat    1696
Asn Pro Phe Glu Ile Gln Ala Asn His Lys Asp Glu Glu Thr Glu Asp
            525                 530                 535

gtc tta gct tct aat ggg tat gaa tct gat gaa caa gaa aag agt gcc    1744
Val Leu Ala Ser Asn Gly Tyr Glu Ser Asp Glu Gln Glu Lys Ser Ala
        540                 545                 550

tat caa gag tat gac agt gac agt gat gtt cct gag gaa ctc aaa cga    1792
Tyr Gln Glu Tyr Asp Ser Asp Ser Asp Val Pro Glu Glu Leu Lys Arg
    555                 560                 565

gac tat gtg gat gag cag aca gga gat ggt cct gtg aaa agt gtt tct    1840
Asp Tyr Val Asp Glu Gln Thr Gly Asp Gly Pro Val Lys Ser Val Ser
570                 575                 580                 585

cgg gaa act cta aaa agc agg aag aaa tca gat tac agt cta aat aaa    1888
Arg Glu Thr Leu Lys Ser Arg Lys Lys Ser Asp Tyr Ser Leu Asn Lys
                590                 595                 600

gtg aat gca cct atc tta aca aat aca aca ttg aac gtc ata aga ctt 1936
Val Asn Ala Pro Ile Leu Thr Asn Thr Thr Leu Asn Val Ile Arg Leu
            605                 610                 615

gtt gga aaa tat atg cag atg atg aac att ctt aag cca att gcc ttt 1984
Val Gly Lys Tyr Met Gln Met Met Asn Ile Leu Lys Pro Ile Ala Phe
        620                 625                 630

gat gtt att cat ttc atg tct caa cta ttt gat tat tac ttg tat gca 2032
Asp Val Ile His Phe Met Ser Gln Leu Phe Asp Tyr Tyr Leu Tyr Ala
    635                 640                 645

ata tat acc ttt ttt ggt cgg aat gat tca ttg gaa tca act gga ctc 2080
Ile Tyr Thr Phe Phe Gly Arg Asn Asp Ser Leu Glu Ser Thr Gly Leu
650                 655                 660                 665

ggc ctt agt agt agt aga cta aga aca act cta aac aga ata caa gaa 2128
Gly Leu Ser Ser Ser Arg Leu Arg Thr Thr Leu Asn Arg Ile Gln Glu
                670                 675                 680

agc ctt att gat cta gaa gtt tca gct gat cct act gcc aca ctc aca 2176
Ser Leu Ile Asp Leu Glu Val Ser Ala Asp Pro Thr Ala Thr Leu Thr
            685                 690                 695

gca gca gaa gaa aga aag gag aag gtg cca agt cca cac ctc agt cac 2224

Ala Ala Glu Glu Arg Lys Glu Lys Val Pro Ser Pro His Leu Ser His
        700                 705                 710

cta gtg gtt ttg aca tct ggg gat acg ttg tat ggg ttg gca gaa aga    2272
Leu Val Val Leu Thr Ser Gly Asp Thr Leu Tyr Gly Leu Ala Glu Arg
    715                 720                 725

gtg gta gcc acg gaa tcc ttg gta ttc ttg gct gaa cag ttt gag ttc    2320
Val Val Ala Thr Glu Ser Leu Val Phe Leu Ala Glu Gln Phe Glu Phe
730                 735                 740                 745

ctt cag cca cat ctg gat gct gtg atg cct gca gtc aaa aag ccc ttt    2368
Leu Gln Pro His Leu Asp Ala Val Met Pro Ala Val Lys Lys Pro Phe
                750                 755                 760

ctt cag cag ttc tat tct cag aca gtc tca acc gcc agt gaa cta cgg    2416
Leu Gln Gln Phe Tyr Ser Gln Thr Val Ser Thr Ala Ser Glu Leu Arg
            765                 770                 775

aaa cca att tac tgg att gta gct ggt aaa gcc ctt gat tat gaa cag    2464
Lys Pro Ile Tyr Trp Ile Val Ala Gly Lys Ala Leu Asp Tyr Glu Gln
        780                 785                 790

atg ctg ctt ctc atg gct aat gtg aaa tgg gat gta aaa gaa att atg    2512
Met Leu Leu Leu Met Ala Asn Val Lys Trp Asp Val Lys Glu Ile Met
    795                 800                 805

tca cag cac aac ata tat gta gat gca cta tta aag gaa ttt gag cag 2560
Ser Gln His Asn Ile Tyr Val Asp Ala Leu Leu Lys Glu Phe Glu Gln
810                 815                 820                 825

ttt aac agg agg cta aat gaa gtt tct aag aga gtt cgc ata ccc ttg 2608
Phe Asn Arg Arg Leu Asn Glu Val Ser Lys Arg Val Arg Ile Pro Leu
                830                 835                 840

cct gtg tct aat ata ctt tgg gaa cat tgt ata cga ttg gct aat cga 2656
Pro Val Ser Asn Ile Leu Trp Glu His Cys Ile Arg Leu Ala Asn Arg
            845                 850                 855

act att gta gaa gga tat gcc aat gtc aag aaa tgc agt aat gag ggt 2704
Thr Ile Val Glu Gly Tyr Ala Asn Val Lys Lys Cys Ser Asn Glu Gly
        860                 865                 870

cgt gcc ctg atg caa ttg gat ttt caa cag ttt tta atg aaa ctt gaa 2752
Arg Ala Leu Met Gln Leu Asp Phe Gln Gln Phe Leu Met Lys Leu Glu
    875                 880                 885

aaa cta aca gat att aga ccc att cct gat aaa gaa ttt gta gaa act 2800
Lys Leu Thr Asp Ile Arg Pro Ile Pro Asp Lys Glu Phe Val Glu Thr
890                 895                 900                 905

tat att aaa gct tat tac cta act gag aat gac atg gaa cgg tgg atc 2848

Tyr Ile Lys Ala Tyr Tyr Leu Thr Glu Asn Asp Met Glu Arg Trp Ile
                910                 915                 920

aaa gag cac agg gaa tat tca acg aag cag ctg acc aat ctg gtg aat 2896
Lys Glu His Arg Glu Tyr Ser Thr Lys Gln Leu Thr Asn Leu Val Asn
            925                 930                 935

gtt tgc ctg gga tcc cat atc aat aag aaa gca aga caa aaa ctt cta 2944
Val Cys Leu Gly Ser His Ile Asn Lys Lys Ala Arg Gln Lys Leu Leu
        940                 945                 950

gca gct ata gat gat ata gac aga cct aaa aga taa tgaacacagc 2990
Ala Ala Ile Asp Asp Ile Asp Arg Pro Lys Arg
955 960 965

tctctttcct caacggcatt gatcctcact caatatatat gacctgaaag ccagtttttt 3050

tatgcacttc tgacaactat ctgctaagaa aactttgtgc atgttttttt gactggaaag 3110

tggaaaatat tgaaatgtgt gtggtgttct catgactttt atatgctgtg gtctcttcaa 3170

cttttggtct catttgttgt aatctgaaat gatgttgccg ccttgtcata acaatggtta 3230

tgtgactaca gttatacatt ttacagaaga atgtaccata agtatataat tagaagaaca 3290

gtggcttaat atatgtatgg gaagtttatg gaaaatgaag ttggcacttt tctaccctct 3350

gagcttggtt cttaataagc ataatgtgag ggtgaatatg tagtatctcc taattatgag 3410

cactgcatga gaattaaaaa acacatgtaa gtaaaatagt tgaaaaatca gtatgttctc 3470

tgtttttaaa atgtcaaagt ttatgtcagg gttaatttag ttataacaaa gtgatcataa 3530

tggtgaaatt taataaatat actctagtat gatcagccta aaaaaaaaaa aaaaaaaaaa 3590

aaaaaa 3596

<210> 2

<211> 964

<212> PRT

<213> Homo sapiens

<400> 2

Met Gln Lys Ile Lys Ser Leu Met Thr Arg Gln Gly Leu Lys Ser Pro
1               5                   10                  15

Gln Glu Ser Leu Ser Asp Leu Gly Ala Ile Glu Ser Leu Arg Val Pro
            20                  25                  30

Gly Lys Glu Glu Phe Arg Glu Leu Arg Glu Gln Pro Ser Asp Pro Gln
        35                  40                  45

Ala Glu Gln Glu Leu Ile Asn Ser Ile Glu Gln Val Tyr Phe Ser Val
    50                  55                  60

Asp Ser Phe Asp Ile Val Lys Tyr Glu Leu Glu Lys Leu Pro Pro Val
65                  70                  75                  80
Leu Asn Leu Gln Glu Leu Glu Ala Tyr Arg Asp Lys Leu Lys Gln Gln
                85                  90                  95
Gln Ala Ala Val Ser Lys Lys Val Ala Asp Leu Ile Leu Glu Lys Gln
            100                 105                 110
Pro Ala Tyr Val Lys Glu Leu Glu Arg Val Thr Ser Leu Gln Thr Gly
        115                 120                 125
Leu Gln Leu Ala Ala Val Ile Cys Thr Asn Gly Arg Arg His Leu Asn
    130                 135                 140
Ile Ala Lys Glu Gly Phe Thr Gln Ala Ser Leu Gly Leu Leu Ala Asn
145                 150                 155                 160
Gln Arg Lys Arg Gln Leu Leu Ile Gly Leu Leu Lys Ser Leu Arg Thr
                165                 170                 175
Ile Lys Thr Leu Gln Arg Thr Asp Val Arg Leu Ser Glu Met Leu Glu
            180                 185                 190
Glu Glu Asp Tyr Pro Gly Ala Ile Gln Leu Cys Leu Glu Cys Gln Lys
        195                 200                 205
Ala Ala Ser Thr Phe Lys His Tyr Ser Cys Ile Ser Glu Leu Asn Ser
    210                 215                 220
Lys Leu Gln Asp Thr Leu Glu Gln Ile Glu Glu Gln Leu Asp Val Ala
225                 230                 235                 240
Leu Ser Lys Ile Cys Lys Asn Phe Asp Ile Asn His Tyr Thr Lys Val
                245                 250                 255
Gln Gln Ala Tyr Arg Leu Leu Gly Lys Thr Gln Thr Ala Met Asp Gln
            260                 265                 270

Leu His Met His Phe Thr Gln Ala Ile His Asn Thr Val Phe Gln Val
        275                 280                 285
Val Leu Gly Tyr Val Glu Leu Cys Ala Gly Asn Thr Asp Thr Lys Phe
    290                 295                 300
Gln Lys Leu Gln Tyr Lys Asp Leu Cys Thr His Val Thr Pro Asp Ser
305                 310                 315                 320
Tyr Ile Pro Cys Leu Ala Asp Leu Cys Lys Ala Leu Trp Glu Val Met
                325                 330                 335
Leu Ser Tyr Tyr Arg Thr Met Glu Trp His Glu Lys His Asp Asn Glu
            340                 345                 350
Asp Thr Ala Ser Ala Ser Glu Gly Ser Asn Met Ile Gly Thr Glu Glu
        355                 360                 365
Thr Asn Phe Asp Arg Gly Tyr Ile Lys Lys Lys Leu Glu His Gly Leu
    370                 375                 380
Thr Arg Ile Trp Gln Asp Val Gln Leu Lys Val Lys Thr Tyr Leu Leu
385                 390                 395                 400
Gly Thr Asp Leu Ser Ile Phe Lys Tyr Asp Asp Phe Ile Phe Val Leu
                405                 410                 415
Asp Ile Ile Ser Arg Leu Met Gln Val Gly Glu Glu Phe Cys Gly Ser
            420                 425                 430
Lys Ser Glu Val Leu Gln Glu Ser Ile Arg Lys Gln Ser Val Asn Tyr
        435                 440                 445
Phe Lys Asn Tyr His Arg Thr Arg Leu Asp Glu Leu Arg Met Phe Leu
    450                 455                 460
Glu Asn Glu Thr Trp Glu Leu Cys Pro Val Lys Ser Asn Phe Ser Ile
465                 470                 475                 480

Leu Gln Leu His Glu Phe Lys Phe Met Glu Gln Ser Arg Ser Pro Ser
                485                 490                 495
Val Ser Pro Ser Lys Gln Pro Val Ser Thr Ser Ser Lys Thr Val Thr
            500                 505                 510
Leu Phe Glu Gln Tyr Cys Ser Gly Gly Asn Pro Phe Glu Ile Gln Ala
        515                 520                 525
Asn His Lys Asp Glu Glu Thr Glu Asp Val Leu Ala Ser Asn Gly Tyr
    530                 535                 540
Glu Ser Asp Glu Gln Glu Lys Ser Ala Tyr Gln Glu Tyr Asp Ser Asp
545                 550                 555                 560
Ser Asp Val Pro Glu Glu Leu Lys Arg Asp Tyr Val Asp Glu Gln Thr
                565                 570                 575
Gly Asp Gly Pro Val Lys Ser Val Ser Arg Glu Thr Leu Lys Ser Arg
            580                 585                 590
Lys Lys Ser Asp Tyr Ser Leu Asn Lys Val Asn Ala Pro Ile Leu Thr
        595                 600                 605
Asn Thr Thr Leu Asn Val Ile Arg Leu Val Gly Lys Tyr Met Gln Met
    610                 615                 620
Met Asn Ile Leu Lys Pro Ile Ala Phe Asp Val Ile His Phe Met Ser
625                 630                 635                 640
Gln Leu Phe Asp Tyr Tyr Leu Tyr Ala Ile Tyr Thr Phe Phe Gly Arg
                645                 650                 655
Asn Asp Ser Leu Glu Ser Thr Gly Leu Gly Leu Ser Ser Ser Arg Leu
            660                 665                 670
Arg Thr Thr Leu Asn Arg Ile Gln Glu Ser Leu Ile Asp Leu Glu Val
        675                 680                 685

Ser Ala Asp Pro Thr Ala Thr Leu Thr Ala Ala Glu Glu Arg Lys Glu
    690                 695                 700
Lys Val Pro Ser Pro His Leu Ser His Leu Val Val Leu Thr Ser Gly
705                 710                 715                 720
Asp Thr Leu Tyr Gly Leu Ala Glu Arg Val Val Ala Thr Glu Ser Leu
                725                 730                 735
Val Phe Leu Ala Glu Gln Phe Glu Phe Leu Gln Pro His Leu Asp Ala
            740                 745                 750
Val Met Pro Ala Val Lys Lys Pro Phe Leu Gln Gln Phe Tyr Ser Gln
        755                 760                 765
Thr Val Ser Thr Ala Ser Glu Leu Arg Lys Pro Ile Tyr Trp Ile Val
    770                 775                 780
Ala Gly Lys Ala Leu Asp Tyr Glu Gln Met Leu Leu Leu Met Ala Asn
785                 790                 795                 800
Val Lys Trp Asp Val Lys Glu Ile Met Ser Gln His Asn Ile Tyr Val
                805                 810                 815
Asp Ala Leu Leu Lys Glu Phe Glu Gln Phe Asn Arg Arg Leu Asn Glu
            820                 825                 830
Val Ser Lys Arg Val Arg Ile Pro Leu Pro Val Ser Asn Ile Leu Trp
        835                 840                 845
Glu His Cys Ile Arg Leu Ala Asn Arg Thr Ile Val Glu Gly Tyr Ala
    850                 855                 860
Asn Val Lys Lys Cys Ser Asn Glu Gly Arg Ala Leu Met Gln Leu Asp
865                 870                 875                 880
Phe Gln Gln Phe Leu Met Lys Leu Glu Lys Leu Thr Asp Ile Arg Pro
                885                 890                 895

Ile Pro Asp Lys Glu Phe Val Glu Thr Tyr Ile Lys Ala Tyr Tyr Leu
            900                 905                 910
Thr Glu Asn Asp Met Glu Arg Trp Ile Lys Glu His Arg Glu Tyr Ser
        915                 920                 925
Thr Lys Gln Leu Thr Asn Leu Val Asn Val Cys Leu Gly Ser His Ile
    930                 935                 940
Asn Lys Lys Ala Arg Gln Lys Leu Leu Ala Ala Ile Asp Asp Ile Asp
945                 950                 955                 960
Arg Pro Lys Arg

<210> 3
<211> 17
<212> DNA
<213> Artificial Sequence

<220>
<223> Description of Artificial Sequence:"GT15A", an
artificially synthesized primer sequence

<400> 3
gttttttttt tttttta 17

<210> 4
<211> 17

<212> DNA

<213> Artificial Sequence

<220>

<223> Description of Artificial Sequence:"GT15C", an

artificially synthesized primer sequence

<400> 4

gttttttttt ttttttc 17

<210> 5

<211> 17

<212> DNA

<213> Artificial Sequence

<220>

<223> Description of Artificial Sequence:"GT15G", an

artificially synthesized primer sequence

<400> 5

gttttttttt ttttttg 17

<210> 6

<211> 184

<212> DNA

<213> Homo sapiens

<400> 6

gggggagaca agcaaataga taattgcaga ggagtagagg cacaacagag ttcagctagg 60

agaggatgtg gagtaggtag aaatgtattc aggatctttt agagttggcg attaattttt 120

ttaagaaaga ttacagtttt atggggtgag ttgttgagta gaggttgggg acaaggatac 180

tgat 184

<210> 7

<211> 10

<212> DNA

<213> Artificial Sequence

<220>

<223> Description of Artificial Sequence:"AG00103", an

artificially synthesized primer sequence

<400> 7

tgacctgagt 10

<210> 8

<211> 1921

<212> DNA

<213> Homo sapiens

<400> 8

gccatggcca attccaatta aatcaccgtt tttgggtgga ggcaagtgtc agtaatattt 60

agagattccc tggtaattcc agtgtagagc caggtttggg aaccaatggc caagatgaga 120

atattatctc aagaatggag caacaggaaa tagaggaact gttctaaaaa attgtttgga 180

agttttactg agaatttttg tagaataaga tagaatgacc taaaatttca ttcttcgaaa 240

tggattctgt tatgtttcaa attagtggga ttttcatgca gagttaaaga tataagaaat 300

taggtagtat tttctaagtt tgttaatcca tttaaacatg aaaggtattt tacttaattg 360

gttacaagaa ttaagaatac aactagtatt tgttataata tatgatagta gagccacaac 420

gttattagtt atagaactta tttggactgt gtttgagagt atttgttgaa ccctgattaa 480

aaagtaaaaa taccagtatt cagaacctta taaaatctac atccagcctt tattaatata 540

cactttccat agtacttcca cagtggcaca atgtgaacct gaaaaagagt attgatacta 600

atattgtttt acgagttttt agtgaagcat tgcctcctca tagtattctt taatgataag 660

gggttttttt tggcctcaat ttaattaact cataaatagt aatgggattc agctgtcttt 720

acttttacat gattggttta cctttgattt tttttcgtga aagaaaaata taggatgagg 780

tgacaaaata aatgtctgtt gcagtcaaca tttgataata taattgttta aggataagaa 840

ataatttttt tatacttcat ccattcaaca aatatttatt gagtacctgc ctactctgaa 900

gcatttctac ttatataaat tctaggaatt tgagcagaat gttttctaga gtttctagta 960

cttctgtgaa atcagataaa ccgatttgac ttgacttctt aaaatccatt aaaagaatcc 1020

taaaaccacc tgctaactgc agagcttggt ttacagtttt accaaaattt gtggagagtg 1080

tgcttgaatc acagtcaaga gacataggtt gaagtattga cttaaacata cagcttgttg 1140

aaaggaagtc attttaatgc tttgtgcctc agtttcttca tttgtgaaaa gatgctattg 1200

aactagactt ttttggaatc ctttttgctc gaatgtttta tactttctat attgtaattt 1260

ttgtaggaga aatatgacac aagaaatata cattctaaat aattttttga gcagtttttg 1320

ttagaagatt tgcatattgg tgtctgtatt agagaggatt agtattgagg tttatgacta 1380

ggtgaattta tttagtgatc atgataaaga ctttcaattt aggattatgt atcatttagg 1440

acaggtgtct tctgcaggtg gtactaaggg agactctaac tatggatctg caaaatcatt 1500

taagagtttt tcttaggtgc tcatggctaa cagtatttag ttgtgttctt tttaaccatt 1560

aatgaaatta tgcttctcat agcttcagat gctttgagat tcttaaccct tctttcatag 1620

caaatcatat aagttcattc atacagtcca tttggcaaac acttatggaa ccaggcagtt 1680

actaatcatt gaagatgcag agaggtacaa gactgttcct aactcaacct gagtggggga 1740

gacaagcaaa aatagataat tgcagaggag tagaggcaca acagagttca gctaggagag 1800

gatgtggagt aggtagaaat gtattcagga tcttttagag ttggcgatta atttttttaa 1860

gaaagattac agttttatgg ggtgagttgt tgagtagagg ttggggacaa ggatactgat 1920

t 1921

<210> 9

<211> 28

<212> DNA

<213> Artificial Sequence

<220>

<223> Description of Artificial Sequence:"1153-2R", an

artificially synthesized primer sequence

<400> 9

aacctctact caacaactca ccccataa 28

<210> 10

<211> 199

<212> DNA

<213> Homo sapiens

<400> 10

gcctcagtga tcttggtgcc atagagagtc tccgggtccc tggaaaggaa gaattcaggg 60

aacttcgaga acagccaagt gaccctcaag ctgaacaaga gcttattaat agtattgaac 120

aagtatattt ttctgtggat tcatttgata ttgttaaata tgagctggag aagcttccac 180

ctgttctcaa tttgcaaga 199

<210> 11

<211> 17

<212> DNA

<213> Artificial Sequence

<220>

<223> Description of Artificial Sequence:"1153-143U17",

an artificially synthesized primer sequence

<400> 11

gaaaagccct caagaaa 17

<210> 12

<211> 21

<212> DNA

<213> Artificial Sequence

<220>

<223> Description of Artificial Sequence:"1153-359L21",

      an artificially synthesized primer sequence

<400> 12

ttgtctctat acgcctctaa t                                                21

<210> 13

<211> 23

<212> DNA

<213> Artificial Sequence

<220>

<223> Description of Artificial Sequence:"1153EST-F", an

      artificially synthesized primer sequence

<400> 13

gggtcatttg tgtagtggct cgg                                              23

<210> 14

<211> 25

<212> DNA
<213> Artificial Sequence

<220>
<223> Description of Artificial Sequence:"1153EST-R", an
      artificially synthesized primer sequence

<400> 14
cctcctccag catttcactt aaccg                                          25

<210> 15
<211> 18
<212> DNA
<213> Artificial Sequence

<220>
<223> Description of Artificial
      Sequence:"1153-3'-207U18", an artificially
      synthesized primer sequence

<400> 15
gagaacagcc aagtgacc                                                  18

<210> 16

<211> 21

<212> DNA

<213> Artificial Sequence

<220>

<223> Description of Artificial

Sequence:"1153-3'-896L21", an artificially

synthesized primer sequence

<400> 16

tttttccaag aagtcgataa g 21.

<210> 17

<211> 636

<212> DNA

<213> Homo sapiens

<400> 17

ctcaagctga acaagagctt attaatagta ttgaacaagt atatttttct gtggattcat 60

ttgatattgt taaatatgag ctgagagagc ttccacctgt tctcaatttg caagaattag 120

aggcgtatag agacaaattg aaacaacagc aagctgcagt atctaaaaaa gtggcagatt 180

taatccttga aaaacagcct gcttatgtaa aggaacttga aagagttacc tcattgcaga 240

caggtcttca attagctgct gttatctgta caaatgggag aagacacttg aatattgcaa 300

aggaaggttt tactcaagct agtttaggcc ttcttgcaaa tcaaaggaaa cgtcagttgc 360

tgattggact tctgaaatct ctgagaacta taaaaacatt gcaaagaaca gatgtacggt 420

taagtgaaat gctggaggag gaagattatc caggagctat tcagttgtgc cttgaatgtc 480

aaaaagctgc cagcactttt aaacattaca gttgtataag tgaactgaat tcaaagctgc 540

aagatacttt ggaacagatt gaggaacagc tggacgtagc tctttccaaa atctgcaaga 600

attttgacat taaccattat accaaggttc aacaag 636

<210> 18

<211> 21

<212> DNA

<213> Artificial Sequence

<220>

<223> Description of Artificial Sequence:"1153-F2", an

artificially synthesized primer sequence

<400> 18

aaagccctca agaaagcctc a 21

<210> 19

<211> 21

<212> DNA

<213> Artificial Sequence

<220>

<223> Description of Artificial Sequence:"1153-R2", an

artificially synthesized primer sequence

<400> 19
ggtcacttgg ctgttctcga a                                                21

<210> 20
<211> 28
<212> DNA
<213> Artificial Sequence

<220>
<223> Description of Artificial Sequence:an artificially
      synthesized TaqMan probe sequence

<220>
<221> misc_binding
<222> (1)
<223> Label FAM (6-carboxy-fluorescein)

<220>
<221> misc_binding
<222> (28)
<223> Label TAMRA
      (6-carboxy-N,N,N',N'-tetramethylrhodamine)

<400> 20
tgatcttggt gccatagaga gtctccgg                                    28

<210> 21
<211> 25
<212> DNA
<213> Artificial Sequence

<220>
<223> Description of Artificial Sequence:an artificially
      synthesized primer sequence

<400> 21
tcacccacac tgtgcccatc tacga                                       25

<210> 22
<211> 25
<212> DNA
<213> Artificial Sequence

<220>
<223> Description of Artificial Sequence:an artificially
      synthesized primer sequence

<400> 22
cagcggaacc gctcattgcc aatgg                                          25

<210> 23
<211> 26
<212> DNA
<213> Artificial Sequence

<220>
<223> Description of Artificial Sequence:an artificially
      synthesized TaqMan probe sequence

<220>
<221> misc_binding
<222> (1)
<223> Label FAM (6-carboxy-fluorescein)

<220>
<221> misc_binding
<222> (7)
<223> Label TAMRA
      (6-carboxy-N,N,N',N'-tetramethylrhodamine)

<400> 23
atgccctccc ccatgccatc ctgcgt                                         26

<210> 24

<211> 284

<212> DNA

<213> Artificial Sequence

<220>

<223> Description of Artificial Sequence:an artificially

synthesized probe sequence

<400> 24

atctctcatg acccgacagg gtctgaaaag ccctcaagaa agcctcagtg atcttggtgc 60

catagagagt ctccgggtcc ctggaaagga agaattcagg gaacttcgag aacagccaag 120

tgaccctcaa gctgaacaag agcttattaa tagtattgaa caagtatatt tttctgtgga 180

ttcatttgat attgttaaat atgagctgga gaagcttcca cctgttctca atttgcaaga 240

attagaggcg tatagagaca aattgaaaca acagcaagct gcag 284

<210> 25

<211> 824

<212> DNA

<213> Homo sapiens

<220>

<221> CDS

<222> (50)..(823)

<400> 25

tgtgatttgt tagctctttg aggcagggta ccctcctcag gatttcgat atg caa aaa 58

Met Gln Lys

1

atc aaa tct ctc atg acc cga cag ggt ctg aaa agc cct caa gaa agc 106

Ile Lys Ser Leu Met Thr Arg Gln Gly Leu Lys Ser Pro Gln Glu Ser

    5                   10                  15

ctc agt gat ctt ggt gcc ata gag agt ctc cgg gtc cct gga aag gaa 154

Leu Ser Asp Leu Gly Ala Ile Glu Ser Leu Arg Val Pro Gly Lys Glu

20                  25                  30                  35

gaa ttc agg gaa ctt cga gaa cag cca agt gac cct caa gct gaa caa 202

Glu Phe Arg Glu Leu Arg Glu Gln Pro Ser Asp Pro Gln Ala Glu Gln

                40                  45                  50

gag ctt att aat agt att gaa caa gta tat ttt tct gtg gat tca ttt 250

Glu Leu Ile Asn Ser Ile Glu Gln Val Tyr Phe Ser Val Asp Ser Phe

            55                  60                  65

gat att gtt aaa tat gag ctg gag aag ctt cca cct gtt ctc aat ttg 298

Asp Ile Val Lys Tyr Glu Leu Glu Lys Leu Pro Pro Val Leu Asn Leu

        70                  75                  80

caa gaa tta gag gcg tat aga gac aaa ttg aaa caa cag caa gct gca 346
Gln Glu Leu Glu Ala Tyr Arg Asp Lys Leu Lys Gln Gln Gln Ala Ala
    85                  90                  95

gta tct aaa aaa gtg gca gat tta atc ctt gaa aaa cag cct gct tat 394
Val Ser Lys Lys Val Ala Asp Leu Ile Leu Glu Lys Gln Pro Ala Tyr
100                 105                 110                 115

gta aag gaa ctt gaa aga gtt acc tca ttg cag aca ggt ctt caa tta 442
Val Lys Glu Leu Glu Arg Val Thr Ser Leu Gln Thr Gly Leu Gln Leu
                120                 125                 130

gct gct gtt atc tgt aca aat ggg aga aga cac ttg aat att gca aag 490
Ala Ala Val Ile Cys Thr Asn Gly Arg Arg His Leu Asn Ile Ala Lys
            135                 140                 145

gaa ggt ttt act caa gct agt tta ggc ctt ctt gca aat caa agg aaa 538
Glu Gly Phe Thr Gln Ala Ser Leu Gly Leu Leu Ala Asn Gln Arg Lys
        150                 155                 160

cgt cag ttg ctg att gga ctt ctg aaa tct ctg aga act ata aaa aca 586
Arg Gln Leu Leu Ile Gly Leu Leu Lys Ser Leu Arg Thr Ile Lys Thr
    165                 170                 175

ttg caa aga aca gat gta cgg tta agt gaa atg ctg gag gag gaa gat 634

Leu Gln Arg Thr Asp Val Arg Leu Ser Glu Met Leu Glu Glu Glu Asp
180                 185                 190                 195

tat cca gga gct att cag ttg tgc ctt gaa tgt caa aaa gct gcc agc     682
Tyr Pro Gly Ala Ile Gln Leu Cys Leu Glu Cys Gln Lys Ala Ala Ser
                200                 205                 210

act ttt aaa cat tac agt tgt ata agt gaa ctg aat tca aag ctg caa     730
Thr Phe Lys His Tyr Ser Cys Ile Ser Glu Leu Asn Ser Lys Leu Gln
            215                 220                 225

gat act ttg gaa cag att gag gaa cag ctg gac gta gct ctt tcc aaa     778
Asp Thr Leu Glu Gln Ile Glu Glu Gln Leu Asp Val Ala Leu Ser Lys
        230                 235                 240

atc tgc aag aat ttt gac att aac cat tat acc aag gtt caa caa g       824
Ile Cys Lys Asn Phe Asp Ile Asn His Tyr Thr Lys Val Gln Gln
    245                 250                 255

<210> 26

<211> 258

<212> PRT

<213> Homo sapiens

<400> 26

Met Gln Lys Ile Lys Ser Leu Met Thr Arg Gln Gly Leu Lys Ser Pro
1               5                   10                  15

Gln Glu Ser Leu Ser Asp Leu Gly Ala Ile Glu Ser Leu Arg Val Pro
                20                  25                  30

Gly Lys Glu Glu Phe Arg Glu Leu Arg Glu Gln Pro Ser Asp Pro Gln
            35                  40                  45

Ala Glu Gln Glu Leu Ile Asn Ser Ile Glu Gln Val Tyr Phe Ser Val
    50                  55                  60

Asp Ser Phe Asp Ile Val Lys Tyr Glu Leu Glu Lys Leu Pro Pro Val
65                  70                  75                  80

Leu Asn Leu Gln Glu Leu Glu Ala Tyr Arg Asp Lys Leu Lys Gln Gln
                85                  90                  95

Gln Ala Ala Val Ser Lys Lys Val Ala Asp Leu Ile Leu Glu Lys Gln
                100                 105                 110

Pro Ala Tyr Val Lys Glu Leu Glu Arg Val Thr Ser Leu Gln Thr Gly
            115                 120                 125

Leu Gln Leu Ala Ala Val Ile Cys Thr Asn Gly Arg Arg His Leu Asn
    130                 135                 140

Ile Ala Lys Glu Gly Phe Thr Gln Ala Ser Leu Gly Leu Leu Ala Asn
145                 150                 155                 160
Gln Arg Lys Arg Gln Leu Leu Ile Gly Leu Leu Lys Ser Leu Arg Thr
                165                 170                 175
Ile Lys Thr Leu Gln Arg Thr Asp Val Arg Leu Ser Glu Met Leu Glu
            180                 185                 190
Glu Glu Asp Tyr Pro Gly Ala Ile Gln Leu Cys Leu Glu Cys Gln Lys
        195                 200                 205
Ala Ala Ser Thr Phe Lys His Tyr Ser Cys Ile Ser Glu Leu Asn Ser
    210                 215                 220
Lys Leu Gln Asp Thr Leu Glu Gln Ile Glu Glu Gln Leu Asp Val Ala
225                 230                 235                 240
Leu Ser Lys Ile Cys Lys Asn Phe Asp Ile Asn His Tyr Thr Lys Val
                245                 250                 255
Gln Gln

## Description

### Technical Field

The present invention relates to genes associated with allergic disease, a method of testing for allergic disease and methods of screening for compounds that serve as candidate therapeutic agents against allergic disease using the expression of the genes as an index.

### Background Art

Allergic diseases such as atopic dermatitis are considered to be multifactorial diseases. These diseases are caused by the interaction of many different genes, whose expressions are influenced by several various environmental factors. Thus, determination of specific genes causing a specific disease has been extremely difficult for allergic diseases.

Additionally, expression of mutated or defective genes, or overexpression or reduced expression of specific genes is thought to be involved in allergic diseases. To elucidate the role of gene expression in diseases, it is necessary to understand how a gene is involved in triggering disease onset and how the expression of the gene is altered by external stimulants such as drugs.

Recent developments in gene expression analysis techniques have enabled analysis and comparison of gene expression of many clinical samples. Among these methods, the differential display (DD) method is useful. The differential display method was originally developed by Liang and Pardee in 1992 (Science, 1992, 257: 967-971). According to this method, different samples of several tens or more can be screened at one time to detect genes whose expressions are different among the samples. Important information to reveal the causative gene of a disease is expected by examining genes with mutations or genes whose expression changes depending on time and environment. Such genes include those whose expression is influenced by environmental factors.

In recent diagnosis of allergic diseases, generally, history taking, and confirmation of family history and anamnesis of the patient are important factors. Further, methods of diagnosing allergy based on more objective information include a method wherein patient's blood sample are tested and method of observing patient's immune response to allergen. Examples of the former method are the allergen-specific IgE measurement, leukocyte histamine release test, lymphocyte stimulating test, and so on. The presence of allergen-specific IgE verifies the allergic reaction against the allergen. However, allergen-specific IgE is not always detected in every patient. Furthermore, the principle of IgE assay requires performing tests for all of the allergens necessary for diagnosis. Alternatively, the leukocyte histamine release test and lymphocyte stimulating test are methods for observing the reaction of the immune system toward a specific allergen *in vitro.* These methods require complicated operation.

Alternatively, another method wherein the immune response observed in a patient actually contacted with an allergen is utilized in diagnosing allergy is also known (latter method). Such tests include the prick test, scratch test, patch test, intradermal reaction, and induction test. These tests allow direct diagnosis of patient's allergic reaction, but can be regarded as high invasive tests wherein patients are actually exposed to allergen.

In addition, regardless of the allergen types, methods to testify the involvement of allergic reaction are also attempted. For example, a high serum IgE titer indicates the occurrence of allergic reaction in a patient. The serum IgE titer is the information corresponding to the total amount of allergen-specific IgE. Though it is easy to determine the total amount of IgE regardless of the type of allergen, IgE titer may be reduced in some patients with non-atopic bronchitis and such.

The number of eosinophils and ECP (eosinophil cationic protein) value are items for diagnosing delayed-type reaction following Type I allergy and allergic inflammatory reaction. The number of eosinophils is considered to reflect the advance of allergic symptoms. ECP, a protein contained in eosinophil granules, is also strongly activated in relation to seizures of asthma patients. Even though these diagnostic items reflect allergy symptoms, the scope thereof usable as the diagnostic barometer is limited.

Therefore, diagnostic indicators, regardless of the type of allergen, useful in comprehending pathological conditions of allergic disease patients and for determining the treatment regimen for the disease have been intensely desired in the art. Furthermore, markers for allergic disease that are less harmful to patients and easily provide information required for diagnosis will be of great use.

### Disclosure of the Invention

An objective of the present invention is to provide genes associated with allergic disease. Another objective of the invention is to provide a method of testing for allergic disease and a method of screening for compounds that serve as candidate therapeutic agents for allergic disease using the expression of the genes of the present invention as an index.

Based on a previously established technique, the "Fluorescent differential display method (Fluorescent DD method)" (T. Ito et al. 1994, FEBS Lett. 351: 231-236), the present inventors developed a new DD system wherein T-cell RNA samples prepared from multiple human blood samples can be analyzed (WO 00/65046). The present inventors applied the DD system to the isolation of genes whose expression level is altered in an allergic disease-specific manner.

Specifically, first, the present inventors measured IgE titers against mite antigen in multiple subjects including normal healthy individuals and patients with allergic diseases (bronchial asthma and atopic dermatitis). The results revealed significantly higher IgE titer scores in the allergic disease patient group (hereinafter abbreviated as "patient group" in some cases) than the normal healthy group, confirming the patient group being allergic to mite antigen.

Then, the present inventors divided multiple subjects into normal healthy group and allergic disease patient group, collected T-cells from blood samples of the subjects, and screened genes whose expression level differ between the two groups using the DD system. As a result, the present inventors succeeded in isolating a gene, "B1153" that showed significantly higher expression levels in the patient group. Since no nucleotide sequence identical to this gene could not be detected in the publicized databases, it was considered to be a novel gene. Furthermore, the present inventors found it possible to test for an allergic disease, and screen for a candidate compound for a therapeutic agent for an allergic disease using the expression level of this gene as an index, accomplishing this invention.

That is, the present invention relates to a gene that shows a high level expression in a subject having an allergic diathesis, a protein encoded by the gene, and their applications. More specifically, this invention relates to a method of testing for an allergic disease using the expression of the gene as an index, a method of detecting the effect of a candidate compound on the expression of the gene, and, furthermore, a method of screening for a candidate compound for a therapeutic agent for an allergic disease based on this detection method.
[1] A method of testing for an allergic disease, said method comprising the following steps of:
   a) measuring the expression level of a gene having the nucleotide sequence of SEQ ID NO: 1 in T-cells of a subject; and
   b) comparing the expression level of the gene with that in T-cells of a normal healthy subject;
[2] The testing method according to [1], wherein the allergic disease is atopic dermatitis;
[3] The testing method according to [1], wherein the gene expression level is measured by PCR of the cDNA of the gene;
[4] The testing method according to [1], wherein the gene expression level is measured by detecting a protein encoded by the gene;
[5] A reagent for testing for an allergic disease, said reagent comprising an oligonucleotide that has a nucleotide sequence complementary to the polynucleotide sequence of SEQ ID NO: 1 or to a complementary strand thereof and that is at least 15 nucleotides long;
[6] A reagent for testing for an allergic disease, said reagent comprising an antibody that recognizes a peptide comprising the amino acid sequence of SEQ ID NO: 2;
[7] A method of detecting the effect of a candidate compound on the expression level of a polynucleotide according to any one of the following (a) through (d) , said method comprising the following steps of:
   (1) contacting a candidate compound with a cell that expresses a polynucleotide of any one of the following (a) through (d),
      (a) a polynucleotide comprising the coding region of the nucleotide sequence of SEQ ID NO: 1,
      (b) a polynucleotide encoding a protein comprising the amino acid sequence of SEQ ID NO: 2,
      (c) a polynucleotide encoding a protein comprising the amino acid sequence of SEQ ID NO: 2 in which one or more amino acids are substituted, deleted, inserted and/or added and which is functionally equivalent to the protein whose expression level is increased in T-cells of an allergic disease patient, and
      (d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient; and
   (2) measuring the expression level of the polynucleotide according to any one of the above-described (a) through (d);
[8] The method according to [7], wherein said cell is a T cell line;
[9] A method of detecting the effect of a candidate compound on the expression level of a polynucleotide according to any one of the following (a) through (d):
   (a) a polynucleotide comprising the coding region of the nucleotide sequence of SEQ ID NO: 1,
   (b) a polynucleotide encoding a protein comprising the amino acid sequence of SEQ ID NO: 2,
   (c) a polynucleotide encoding a protein comprising the amino acid sequence of SEQ ID NO: 2 in which one or more amino acids are substituted, deleted, inserted and/or added and that is functionally equivalent to the protein whose expression level is increased in T-cells of an allergic disease patient, and
   (d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient,
   wherein said method comprises the following steps of:
   (1) administering a candidate compound to a test animal, and
   (2) measuring the expression level of the polynucleotide according to any one of the above-described (a) through (d) in T-cells of the test animal;
[10] A method of screening for a compound that reduces the expression level of a polynucleotide according to any one of the above-described (a) through (d), said method comprising the steps of detecting the effect of a candidate compound on said expression level by the method according to [7] or [9], and selecting a compound that reduces said expression level compared to a control;
[11] A method of detecting the effect of a candidate compound on the activity of a transcriptional regulatory region of a gene having the nucleotide sequence of SEQ ID NO: 1, said method comprising the following steps of:
   (1) contacting a candidate compound with a cell into which a vector has been introduced, wherein the vector contains the transcriptional regulatory region of the gene having the nucleotide sequence of SEQ ID NO: 1 and a reporter gene that is expressed under the control of said transcriptional regulatory region, and
   (2) measuring the activity of said reporter gene;
[12] A method of screening for a compound that reduces the activity of the transcriptional regulatory region of the gene having the nucleotide sequence of SEQ ID NO: 1, said method comprising the steps of detecting the effect of a candidate compound on said activity by the method according to [11], and selecting a compound that reduces said activity compared to a control;
[13] A method of detecting the effect of a candidate compound on the activity of a protein encoded by a polynucleotide according to any one of (a) through (d) , said method comprising the following steps of:
   (1) contacting a candidate compound with a protein encoded by a polynucleotide according to any one of the following (a) through (d),
      (a) a polynucleotide comprising the coding region of the nucleotide sequence of SEQ ID NO: 1,
      (b) a polynucleotide encoding a protein comprising the amino acid sequence of SEQ ID NO: 2,
      (c) a polynucleotide encoding a protein comprising the amino acid sequence of SEQ ID NO: 2 in which one or more amino acids are substituted, deleted, inserted and/or added and that is functionally equivalent to the protein whose expression level is increased in T-cells of an allergic disease patient, and
      (d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient; and
   (2) measuring the activity of said protein;
[14] The method according to [13], wherein said activity of a protein is its binding activity to myosin binding subunit 85 or skeletal muscle alpha 2 actinin;
[15] A method of screening for a compound that reduces the activity of a protein encoded by a polynucleotide according to any one of (a) through (d) , said method comprising the steps of detecting the effect of a candidate compound on said activity by the method according to [13], and selecting a compound that reduces said activity compared to a control;
[16] A vector containing a transcriptional regulatory region of a gene having the nucleotide sequence of SEQ ID NO: 1 and a reporter gene that is expressed under the control of said transcriptional regulatory region;
[17] A cell into which the vector according to [16] has been introduced;
[18] A therapeutic agent for an allergic disease, said agent comprising a compound obtainable by the screening method according to any one of claims 10, 12 and 15 as an effective ingredient;
[19] A therapeutic agent for an allergic disease, said agent comprising, as a principal ingredient, an antisense DNA against a polynucleotide having the nucleotide sequence of SEQ ID NO: 1, or a portion thereof;
[20] A therapeutic agent for an allergic disease, said agent comprising, as a principal ingredient, an antibody that binds to a protein having the amino acid sequence of SEQ ID NO: 2;
[21] A polynucleotide according to any one of the following (a) through (d):
   (a) a polynucleotide comprising the coding region of the nucleotide sequence of SEQ ID NO: 1,
   (b) a polynucleotide encoding a protein comprising the amino acid sequence of SEQ ID NO: 2,
   (c) a polynucleotide encoding a protein comprising the amino acid sequence of SEQ ID NO: 2 in which one or more amino acids are substituted, deleted, inserted, and/or added that is functionally equivalent to the protein whose expression level is increased in T-cells of an allergic disease patient, and
   (d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient;
[22] A protein encoded by the polynucleotide according to [21];
[23] A vector harboring the polynucleotide according to [21] in an expressible manner;
[24] A transformed cell harboring the polynucleotide according to [21] or the vector according to [23];
[25] A method of preparing the protein according to [22] , said method comprising the steps of culturing the transformed cells according to [24], and collecting an expression product thereof;
[26] An antibody against the protein according to [22];
[27] A method of immunologically measuring the protein according to [22], said method comprising the step of observing the immunoreaction of the antibody according to [26] with the protein according to [22];
[28] An oligonucleotide that has a nucleotide sequence complementary to the polynucleotide sequence of SEQ ID NO: 1 or to a complementary strand thereof and that is at least 15 nucleotides long;
[29] A method of measuring the polynucleotide according to [21], wherein said method comprising the step of observing hybridization of the oligonucleotide according to [28] to the polynucleotide according to [21];
[30] An allergic disease animal model comprising a transgenic non-human vertebrate in which the expression level of a polynucleotide according to any one of the following (a) through (d) is elevated in its T-cells:
   (a) a polynucleotide comprising the coding region of the nucleotide sequence of SEQ ID NO: 1,
   (b) a polynucleotide encoding a protein comprising the amino acid sequence of SEQ ID NO: 2,
   (c) a polynucleotide encoding a protein comprising the amino acid sequence ser forth in SEQ ID NO: 2 in which one or more amino acids are substituted, deleted, inserted, and/or added and that is functionally equivalent to the protein whose expression level is increased in T-cells of an allergic disease patient, and
   (d) a polynucleotide hybridizing to a DNA comprising a nucleotide sequence of SEQ ID NO: 1 under stringent conditions,
   wherein the polynucleotide encodes a protein whose expression level is increased in T-cells of an allergic disease patient;
[31] A kit for screening for a candidate compound for a therapeutic agent for an allergic disease, said kit comprising a polynucleotide that hybridizes to the nucleotide sequence of SEQ ID NO: 1 or to a complementary sequence thereof and that is at least 15 nucleotides long, and a cell expressing the gene comprising the nucleotide sequence of SEQ ID NO: 1;
[32] A kit for screening for a candidate compound for a therapeutic agent for an allergic disease, said kit comprising an antibody that recognizes a peptide comprising an amino acid sequence selected from that of SEQ ID NO: 2, and a cell expressing the gene comprising the nucleotide sequence of SEQ ID NO: 1;
[33] A kit for screening for a candidate compound for a therapeutic agent for an allergic disease, said kit comprising a protein comprising the amino acid sequence of SEQ ID NO: 2 and a protein that interacts with said protein; and
[34] The kit according to [33], wherein the interacting protein is a protein selected from the group consisting of myosin binding subunit 85, skeletal muscle alpha 2 actinin, and a fragment comprising an interacting domain thereof.

Alternatively, this invention relates to a method of treating an allergic disease comprising the step of administering a compound being obtainable by the screening method according to any one of the above-described [10], [12] and [15]. Furthermore, this invention relates to the use of a compound being obtainable by the screening method according to any one of [10], [12] and [15] in manufacturing a medicinal composition for the treatment of an allergic disease. In addition, this invention relates to a method of treating an allergic disease comprising the step of administering an antisense DNA against the "B1153" gene, or an antibody that binds to the "B1153" protein. Furthermore, this invention relates to the use of an antisense DNA against the "B1153" gene, or an antibody binding to the "B1153" protein in manufacturing a medicinal composition for the treatment of an allergic disease.

This invention relates to a novel "B1153" gene, and a method of testing for an allergic disease using the expression level of "B1153" in T-cells as an index. "B1153" has the nucleotide sequence of SEQ ID NO: 1. Furthermore, "B1153" is a gene whose expression level is varied between the group of normal healthy subjects and that of allergic disease patients. No known gene having a particularly high structural homology to the "B1153" gene according to this invention could be detected by searching the publicized databases.

An approximately 193 bp region on the 5'-side of the "B1153" sequence showed a 96% homology to the 5' EST of a human secretory protein that had been already reported (SEQ ID 20811 in the European Patent No. 1,033,401) but not a complete homology thereto. Accordingly, "B1153" was thought to be a novel gene. Needless to say, there is no report indicating the association of this EST with an allergic disease. Association of "B1153" having the nucleotide sequence of SEQ ID NO: 1 with an allergic disease is the information that has been discovered by the present inventors for the first time now.

"B1153" was isolated as a full-length 3596 bp cDNA clone containing an open reading frame (ORF). The determined nucleotide sequence of "B1153" cDNA and the amino acid sequence encoded by this nucleotide sequence were set forth in SEQ ID NOs: 1 and 2, respectively.

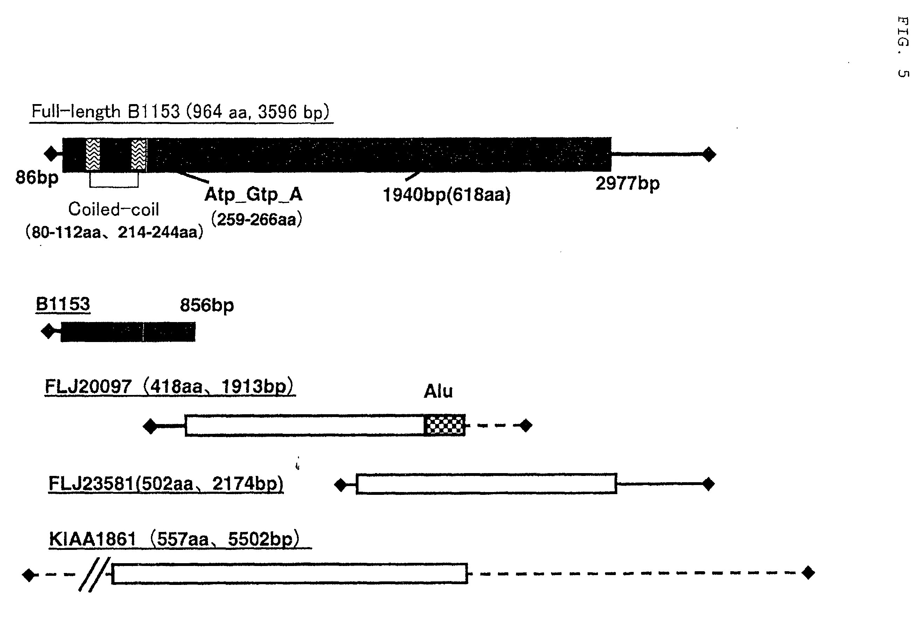

A search of databases for homology of this sequence information revealed that, in the "B1153" gene nucleotide sequence of SEQ ID NO: 1, the sequence from 505 bp to 2148 bp, and that from 1420 bp to 3596 bp were highly homologous to KIAA1861 (Accession No. AB058764) and FLJ2358 (Accession No. AK027234), respectively. However, the sequence from 1 bp to 504 bp was a novel one. Relationships among these nucleotide sequences are summarized in Fig. 5. Except for these nucleotide sequences, no other known amino acid sequence was found to be homologous to this amino acid sequence encoded by the nucleotide sequence, and thus, "B1153" was thought to encode a novel protein.

Nucleotide sequence of SEQ ID NO: 1 is a full-length cDNA. This cDNA can be obtained by screening the T-cell cDNA library using probes selected from the nucleotide sequence of SEQ ID NO: 1.

This invention relates to a polynucleotide comprising the nucleotide sequence of SEQ ID NO: 1. This invention also relates to a polynucleotide that hybridizes under stringent conditions to the polynucleotide comprising the nucleotide sequence of SEQ ID NO: 1 and that encodes a protein functionally equivalent to the protein encoded by the polynucleotide comprising the nucleotide sequence of SEQ ID NO: 1. In this invention, polynucleotide includes, besides a natural nucleic acid molecule such as DNA and RNA, artificial molecules comprising labeled molecule and various nucleotide derivatives. Artificial polynucleotides include polynucleotides having the phosphorothioate bond and peptide bond as a backbone.

These polynucleotides according to this invention can be chemically synthesized, or isolated from the natural nucleic acids such as mRNA, cDNA library, or genomic library. Polynucleotide molecules according to this invention are useful as the probes for detecting the production of protein encoded by them, antisense nucleic acids that inhibit the "B1153" expression, or the presence thereof by hybridization.

In this invention, when the expression level of a protein is increased in T-cells of a patient or an allergic disease animal model, the protein is regarded as being functionally equivalent to the protein of this invention. Increase in the expression level of a protein in T-cells can be confirmed by comparing the expression level of the gene encoding the protein in the collected T-cells.

A polynucleotide that hybridizes under stringent conditions to the polynucleotide comprising the nucleotide sequence of SEQ ID NO: 1 and that encodes a functionally equivalent protein can be obtained by known techniques such as hybridization and PCR based on the nucleotide sequence of SEQ ID NO: 1. For example, by screening the T-cell cDNA library using an oligonucleotide comprising a nucleotide sequence selected from the nucleotide sequence of SEQ ID NO: 1 as a probe, it is possible to obtain cDNA comprising a nucleotide sequence that is highly homologous to that of SEQ ID NO: 1.

When a polynucleotide hybridizes to the polynucleotide comprising the nucleotide sequence of SEQ ID NO: 1 under stringent conditions, in most cases, such a protein encoded by the polynucleotide is thought to have the activity similar to that of the protein of this invention. Stringent conditions mean as follows, that is, hybridization in 4x SSC at 65°c followed by washing with 0.1X SSC at 65°c for 1 hour.

Temperature conditions for hybridization and washing that greatly influence stringency can be adjusted according to the melting temperature (Tm). Tm is varied with the ratio of constitutive nucleotides in the hybridizing base pairs, and the composition of hybridization solution (concentrations of salts, formamide, and sodium dodecyl sulfate). Therefore, considering these conditions, those skilled in the art can put their experience to select an appropriate condition to confer an equal stringency.

A protein encoded by cDNA comprising the nucleotide sequence that has a high identity to the cDNA of this invention is highly likely to be a functionally equivalent protein in this invention. Nucleotide sequence with a high identity in this invention refers to a nucleotide sequence that shows 70% or more homology in general, usually 80% or more, preferably 90% or more, more preferably 95% or more, furthermore preferably 98% or more, and specifically preferably 99% or more identity with the nucleotide sequence of this invention. The degree of identity of one nucleotide sequence to another can be determined by following the well-known algorism BLASTN and such.

Alternatively, by PCR performed using oligonucleotides comprising nucleotide sequences selected from the sequence of SEQ ID NO: 1 as the primers also with the T-cell cDNA library as a template, it is possible to obtain cDNA with a high identity with cDNA of this invention. If human cells are used as the source of cDNA, it is possible to obtain human cDNA. And, when cells from vertebrates other than humans are used, it is possible to obtain the counterpart of human cDNA in different animal species. Examples of such non-human vertebrates are various experimental animals such as mice, rats, dogs, pigs, goats. Counterparts of "B1153" in experimental animals are useful in preparing allergic disease animal models in various animal species and as the marker in developing therapeutic agents for allergic disease.

Alternatively, a gene encoding a protein having, for example, 90% or more, preferably 95% or more, and furthermore preferably 99% or more homology to the amino acid sequence of "B1153" protein can be referred to as a gene functionally equivalent to the "B1153" gene. A gene that can be amplified using, as primers, oligonucleotides comprising nucleotide sequences selected from the sequence according to SEQ ID NO: 1 used in the examples and that encodes a protein that is highly expressed in patients with an allergic diathesis is also a functionally equivalent gene. In this invention, a gene comprising the nucleotide sequence of SEQ ID NO: 1, or a gene functionally equivalent to this gene is referred to as an indicator gene. And, a protein encoded by the indicator gene is termed an indicator protein.

Polynucleotides of this invention include those encoding proteins comprising the amino acid sequence of SEQ ID NO: 2 in which one or a plurality of amino acids are substituted, deleted, added and/or inserted, and which encode proteins functionally equivalent to the protein of this invention. For example, polymorphism is often observed among genes of eukaryotes. In some cases, one or more amino acids may be substituted by polymorphism, but usually the original activity of the protein is retained. It is also known that, even by the modification of amino acid sequence with one or several amino acids, the protein activity is often usually retained. Therefore, all the polynucleotides, which encode proteins whose amino acid sequences are mutated by the modification of one or more amino acids through the artificial modification of the polynucleotide encoding the amino acid sequence of SEQ ID NO: 2, are included in this invention so far as these proteins have functions characteristic to those encoded by the gene of this invention. Preferably, such amino acid sequences include the sequences that have 90% or more homology to the amino acid sequence of SEQ ID NO: 2. The homology of one amino acid sequence can be determined by FASTA.

Herein, codons for respective amino acids are known, and may be arbitrarily selected, and can be determined, for example, according to standard procedures considering the codon use frequency of the host to be employed (Grantham, R. et al. Nucleic Acids Res. 9, r43 (1981)). Therefore, nucleotides whose DNAs are appropriately modified considering the degeneracy of codons are also included in the polynucleotide of this invention. Codons in these nucleotide sequences can be partially modified according to the site-specific mutagenesis method (Mark, D. F. et al., Proc. Natl. Acad. Sci. U.S.A. 81, 5662 (1984)) or such using primers comprising synthetic oligonucleotides that encode the desired modification.

This invention also relates to an oligonucleotide that comprises a nucleotide sequence complementary to the polynucleotide having the nucleotide sequence of SEQ ID NO: 1 or to the complementary strand thereof, and that is at least 15-nucleotide-long. Herein, the term "complementary strand" is defined as one strand of a double stranded polynucleotide composed of A: T (U for RNA) and G:C base pairs to the other strand. In addition, "complementary" can be defined as not only when strands are completely homologous within a region of at least 15 continuous nucleotides, but also when they have at least 70%, preferably at least 80%, more preferably 90%, and even more preferably 95% or higher homology within that region. The degree of homology of one nucleotide sequence to another can be determined by following the algorithm described in this specification.

Oligonucleotides of the present invention are useful for detecting and synthesizing the polynucleotide of this invention. Techniques for detecting or synthesizing the target nucleotide using oligonucleotides as the probe or primer are known. For example, Northern blot technique with mRNA as a target polynucleotide is a typical method of detecting RNA. RT-PCR that is carried out with mRNA as a template enables the synthesis of polynucleotide of this invention. Furthermore, it is also possible to find out the presence of mRNA as well as its expression level using the presence and amount of that synthetic product as an index. Alternatively, the polynucleotide of this invention that is expressed in T-cells can be detected by an *in situ* hybridization technique.

Furthermore, using the polynucleotide of this invention, a protein encoded thereby can be produced as a recombinant. More specifically, a transformant is obtained by inserting the coding region of the polynucleotide having the nucleotide sequence of SEQ ID NO: 1 into a known expression vector, and transfecting the resulting recombinant vector into an appropriate host. Alternatively, a transformant is also obtained by integrating the polynucleotide containing the coding region into a genome of an appropriate host.

By culturing the resulting transformant under the conditions in which the polynucleotide of this invention can be expressed to collect the expression product, the protein of this invention can be obtained. Expression product can be purified by known techniques.

In addition, the present invention also relates to a protein encoded by the polynucleotide of this invention. The protein of this invention is useful as an indicator for diagnosing an allergic disease such as atopic dermatitis.

Additionally, the protein of the present invention and its fragments are useful as the antigen for producing an antibody against the protein of this invention. Techniques for obtaining an antibody using a given antigen are known. That is, a protein or its fragment is mixed with an appropriate adjuvant, and the antigen thus formed is inoculated to an animal to be immunized. There is no limitation in the type of animals to be immunized. Typical examples of animal to be immunized are such as mice, rats, rabbits, goats. After the increase in the antibody titer is confirmed, blood is collected, and the serum is fractionated as an antiserum. Alternatively, by further purifying the IgG fraction, a purified antibody can be obtained. For the purification of antibody, techniques such as ammonium sulfate precipitation, ion exchange chromatography, immunoaffinity chromatography using protein A-conjugated Sepharose and the protein of this invention as the ligand can be utilized.

Furthermore, it is also possible to obtain a monoclonal antibody by transforming an antibody-producing cell using techniques such as cell fusion, and cloning the resulting transformant. Alternatively, a method of isolating a gene of the antibody-producing cell and constructing a humanized antibody and chimeric antibody is also known. Antibody thus obtained is useful as a tool for immunologically measuring the protein of this invention. For the immunoassay according to this invention, a variety of known assay formats can be applied. For example, in the case of a protein contained in serum or such, it can be measured by ELISA or such, or for the detection of a protein expressed in T-cells with antibody, immunohistochemical technique or fluorescence activated cell sorter (FACS) using a fluorescence labeled antibody can be utilized.

In the present invention, the term "allergic disease" is a general term for diseases in which allergic reaction is involved. More specifically, to consider a disease to be allergic, an allergen must be identified, a strong correlation between the exposure to the allergen and the onset of the pathological change must be demonstrated, and the pathological change must be proven to have an immunological mechanism. Herein, an immunological mechanism means that immune responses by the T-cells are induced by the stimulation of the allergen. Examples of allergens include mite antigen, pollen antigen.

Representative allergic diseases include bronchial asthma, allergic rhinitis, atopic dermatitis, pollen allergy, insect allergy, and such. Allergic diathesis is a genetic factor that is inherited from allergic parents to their children. Familial allergic diseases are also called atopic diseases, and the causative factor that is inherited is the atopic diathesis. The term "atopic dermatitis" is a general term for atopic diseases, especially, the diseases with dermatitis.

The "B1153" gene of the present invention showed statistically significant high expression level in the patient group according to the comparison between the normal healthy subject group and allergic disease patient group. Therefore, it is possible to test for allergic disease using the expression level of "B1153" gene as an index.

Tests for allergic disease of the present invention include, for example, those as described below. A test for judging whether an allergic disease-like symptom is caused by allergic reaction can be mentioned. More specifically, allergic disease-like symptoms are exemplified by dermatitis (itching, flare); rhinitis (nasal congestion, running nose, sneeze); asthma (stridor, dyspnea); and so on. Although these symptoms are also observed in xeroderma, cold syndrome (cold in the nose), bronchitis, and such, it is possible to judge whether these symptoms are caused by allergic reaction or not according to the test method of the present invention. In addition, the method of testing for allergic disease of the present invention includes a test to judge whether a subject has allergic diathesis or not.

Herein, the expression level of the "B1153" gene includes the transcription of the gene to mRNA as well as the translation into protein. Therefore, a method for testing for allergic disease according to the present invention is performed by comparing the expression level of mRNA corresponding to the gene, or the expression level of a protein encoded by the gene.

Measurement of the expression level of the "B1153" gene in a test for allergic disease of the present invention may be conducted according to known gene analytical methods. More specifically, for example, a hybridization technique with a nucleic acid that hybridizes to the gene as a probe, a gene amplification technique with a DNA hybridizing to the gene of this invention as a primer, or such can be utilized.

As a primer or probe for the test according to the present invention can be used a polynucleotide comprising the nucleotide sequence selected from that of SEQ ID NO: 1 or at least 15 nucleotides that are complementary to the complementary strand thereof. Herein, the term "complementary strand" means one strand of a double stranded DNA composed of A:T (U for RNA) and G:C base pairs to the other strand. In addition, "complementary" means not only those completely complementary to a region of at least 15 continuous nucleotides, but also having a homology of at least 70%, preferably at least 80%, more preferably 90%, and even more preferably 95% or higher. The degree of homology between nucleotide sequences can be determined by the algorithm such as BLASTN.

Such polynucleotides can be useful as the probe to detect and isolate the polynucleotide encoding the protein according to the present invention, or as the primer to amplify the polynucleotide according to the present invention. When used as a primer, those polynucleotides comprise usually 15 bp to 100 bp, preferably 15 bp to 35 bp of nucleotides. When used as a probe, DNAs comprising the whole sequence of the polynucleotide according to the present invention, or a partial sequence thereof that contains at least 15-bp nucleotides. When used as a primer, the 3' region thereof must be complementary to the indicator gene, while the 5' region can be linked to a restriction enzyme-recognition sequence or tag.

"Polynucleotides" in the present invention may be either DNA or RNA. These polynucleotides may be either synthetic or naturally-occurring. Also, DNA used as a probe for hybridization is usually labeled. Examples of labeling methods are those as described below. Herein, the term "oligonucleotide" means a polynucleotide with relatively low degree of polymerization. Oligonucleotides are included in polynucleotides.
· nick translation labeling using DNA polymerase I;
· end labeling using polynucleotide kinase;
· fill-in end labeling using Klenow fragment (Berger, SL, Kimmel, AR. (1987) Guide to Molecular Cloning Techniques, Method in Enzymology, Academic Press; Hames, BD, Higgins, SJ (1985) Genes Probes: A Practical Approach. IRL Press; Sambrook, J, Fritsch, EF, Maniatis, T. (1989) Molecular Cloning: a Laboratory Manual, 2nd Edn. Cold Spring Harbor Laboratory Press) ;
· transcription labeling using RNA polymerase (Melton, DA, Krieg, PA, Rebagkiati, MR, Maniatis, T, Zinn, K, Green, MR. (1984) Nucleic Acid Res., 12, 7035-7056); and
· non-isotopic labeling of DNA by incorporating modified nucleotides (Kricka, LJ. (1992) Nonisotopic DNA Probing Techniques. Academic Press).

For testing for an allergic disease using hybridization techniques, for example, Northern hybridization, dot blot hybridization, or DNA microarray technique may be used. Furthermore, gene amplification techniques, such as RT-PCR method may be used. By using the PCR amplification monitoring method during the gene amplification step in RT-PCR, one can achieve more quantitative analysis for the gene expression of the present invention.

In the PCR gene amplification monitoring method, the detection target (DNA or reverse transcript of RNA) is hybridized to probes that are dual-labeled at both ends with different fluorescent dyes whose fluorescences cancel each other out. When the PCR proceeds and Taq polymerase degrades the probe with its 5'-3' exonuclease activity, the two fluorescent dyes become distant from each other and the fluorescence becomes to be detected. The fluorescence is detected in real time. By simultaneously measuring a standard sample in which the copy number of the target is known, it is possible to determine the copy number of the target in the subject sample with the cycle number where PCR amplification is linear (Holland, P. M. et al., 1991, Proc. Natl. Acad. Sci. USA 88: 7276-7280; Livak, K. J. et al., 1995, PCR Methods and Applications 4 (6) : 357-362; Heid, C. A. et al., 1996, Genome Research 6: 986-994; Gibson, E. M. U. et al., 1996, Genome Research 6: 995-1001). For the PCR amplification monitoring method, for example, ABI PRISM7700 (PE Biosystems) may be used.

The method of testing for allergic disease of the present invention can be also carried out by detecting a protein encoded by the "B1153" gene. For such test methods, for example, the Western blotting method, the immunoprecipitation method, the ELISA method, and such that utilize antibodies binding to a protein encoded by this gene may be employed.

Antibodies that bind to the "B1153" protein used in the detection may be produced by techniques known to those skilled in the art. Antibodies used in the present invention may be polyclonal or monoclonal antibodies (Milstein, C. et al., 1983, Nature 305 (5934) : 537-40). For example, polyclonal antibody against a protein of the present invention may be produced by collecting blood from mammals sensitized with an antigen, and separating the serum from this blood using known methods. As a polyclonal antibody, the serum containing polyclonal antibody may be used. According to needs, a fraction containing polyclonal antibody can be further isolated from this serum. Alternatively, a monoclonal antibody can be obtained by isolating immune cells from mammals sensitized with an antigen; fusing these cells with myeloma cells, and such; cloning hybridomas thus obtained; and collecting the antibody from the culture as the monoclonal antibody.

To detect the "B1153" protein, these antibodies may be appropriately labeled. Alternatively, instead of labeling the antibody, a substance that specifically binds to antibodies, for example, protein A or protein G, may be labeled to arrange an indirect detection of the proteins. More specifically, one example of an indirect detection method is ELISA.

A protein or partial peptides thereof that is used as an antigen may be obtained, for example, by inserting a gene or portion thereof into an expression vector, introducing it into an appropriate host cell to produce a transformant, culturing the transformant to express the recombinant protein, and purifying the expressed recombinant protein from the culture or the culture supernatant. Alternatively, oligonucleotides consisting of the amino acid sequence encoded by the gene, or partial amino acid sequences of the amino acid sequence encoded by the full-length cDNA of SEQ ID NO: 1 are chemically synthesized to be used as the antigen.

T-cells from subjects are used as the test sample in the present invention. T-cells can be prepared from peripheral blood by known methods. Specifically, for example, heparinized collected blood is fractionated by centrifugation to isolate lymphocytes. The separated lymphocytes may be directly used as the sample for the test for allergic disease of the present invention. Direct analysis of not a purified T-cell fraction but the lymphocyte fraction as a test sample enables a convenient bed-side test. Alternatively, T-cells may be isolated by fractionating CD3-positive cells from separated lymphocytes using CD3 microbeads labeling, followed by separation using a cell sorter, and such. Lysate prepared by disintegrating the separated T-cells may serve as a sample for the immunological assay of the above-described protein. Alternatively, mRNA extracted from this lysate may be used as a sample for measuring mRNA corresponding to the gene. Preparation of T-cell lysate and mRNA extraction may be conveniently carried out using commercially available kits.

Alternatively, the expression level of a gene that serves as the indicator in this invention may be measured using the whole blood, and peripheral blood lymphocyte population as the object without isolating T-cells. In this case, by correcting the measured values, the variance of gene expression levels in cells can be determined. For example, based on the measured value of the expression level of a gene (housekeeping gene) , whose expression level is T-cell specific and is not widely altered regardless of the cellular conditions, the measured value of the expression level of a gene serving as an index in this invention can be corrected.

Alternatively, in the case where the protein to be detected is a secretory protein, comparison of the expression level of a gene encoding the protein is accomplished by measuring the amount of the target protein contained in body fluid sample, such as blood and serum, in a subject.

When the expression level of a gene of the present invention is higher in a subject compared with that in normal healthy individuals as a result of testing for allergic disease according to the present invention, the subject may be determined to suffer allergic disease. Alternatively, in the test for an allergic diathesis, the subject may be judged to have allergic diathesis.

Furthermore, the present invention relates to an allergic disease animal model comprising a non-human transgenic animal with an increased expression level in T-cells of a polynucleotide selected from the group of:
(a) a polynucleotide comprising the coding region of the nucleotide sequence of SEQ ID NO: 1;
(b) a polynucleotide encoding a protein comprising the amino acid sequence of SEQ ID NO: 2;
(c) a polynucleotide encoding a protein comprising the amino acid sequence of SEQ ID NO: 2 in which one or more amino acids are substituted, deleted, inserted and/or added and which is functionally equivalent to the protein whose expression level is increased in T-cells of an allergic disease patient; and
(d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient.

This invention revealed that the expression level of the "B1153" gene in T-cells is elevated in allergic disease patients. Therefore, animal in which the expression level of this gene or a gene functionally equivalent thereto is artificially increased in T-cells can be utilized as an allergic disease animal model. Herein, increase in the expression level of the indicator gene in T-cells includes that increase in blood cells. That is, increase in the expression level of the gene includes not only the case where the increase occurs in T-cells but also the cases where it occurs in the whole blood cells, or systemically in the whole body. In this invention, a functionally equivalent gene means any one of those polynucleotides described in the aforementioned (a) through (d).

For example, if allergic disease animal models according to the present invention either develop clinical manifestations of allergic dermatitis or show changes in measured values related to any allergic diseases, it is possible to construct a screening system for searching for a compound having activity to recover normal conditions.

In the present invention, increase in the expression level means the state wherein a target gene is transduced as a foreign gene and forcibly expressed; the state wherein transcription of a gene inherent in the host and translation thereof into protein are increased; or the state wherein decomposition of the translation product, protein, is suppressed. Gene expression level can be confirmed by, for example, the quantitative PCR as described in Examples. Furthermore, activity of translation product, protein, can be confirmed by comparing to that in the normal state.

A typical transgenic animal is the one to which a gene of interest is transduced to be forcibly expressed. Examples of another type of transgenic animals are those in which a mutation is introduced into the coding region of the gene to increase its activity or to modify the amino acid sequence of the gene product protein so as to be hardly decomposed. Examples of mutation in the amino acid sequence are the substitution, deletion, insertion, or addition of amino acid(s). In addition, by mutagenizing the transcriptional regulatory region of the gene, the expression itself of the gene of this invention can be controlled.

Methods for obtaining transgenic animals with a particular gene as a target are known. That is, a transgenic animal can be obtained by a method wherein the gene and ovum are mixed and treated with calcium phosphate; a method wherein the gene is introduced directly into the nucleus of oocyte in pronuclei with a micropipette under a phase contrast microscope (microinjection method, US Patent No. 4873191); or a method wherein embryonic stem cells (ES cells) are used. Furthermore, there have been developed a method for infecting ovum with a gene-inserted retrovirus vector, a sperm vector method for transducing a gene into ovum via sperm, or such. Sperm vector method is a gene recombination technique for introducing a foreign gene by fertilizing ovum with sperm after a foreign gene has been incorporated into sperm by the adhesion or electroporation method, and so on. (M. Lavitranoet, et al. Cell, 57, 717, 1989).

Transgenic animals of the present invention can be produced using all the vertebrates except for humans. More specifically, transgenic animals having various transgene and being modified gene expression levels thereof are produced using vertebrates such as mice, rats, rabbits, miniature pigs, goats, sheep, or cattle.

Transgenic animals of the present invention are useful in not only screening for drugs for treating or preventing allergic diseases as described below but also are useful for elucidating mechanisms of allergic diseases, as well as testing the safety of the screened compounds.

Furthermore, the present invention relates to a method of detecting the effect of a candidate compound on the expression level of the polynucleotide of this invention. In this invention, the "B1153" gene is expressed in a significantly high level in humans with an allergic disease. Therefore, based on the method of detecting the effect on the expression level of this gene, by selecting a compound that enables to reduce the gene expression level compared to a control, it is possible to obtain a therapeutic agent for an allergic disease. In this invention, a compound that reduces the expression level of a gene is the compound that has an inhibitory action on any step of the transcription and translation of a gene as well as the activity expression of a protein encoded by the gene.

The method of detecting the effect of a candidate compound on the expression level of the polynucleotide of this invention can be carried out either *in vivo* or *in vitro.* For detecting the effect *in vivo,* an appropriate test animal is used. As the test animal, for example, allergic disease animal models, and those comprising transgenic, non-human animals in which the expression of a polynucleotide according to any one of the above-described (a) through (d) in T-cells is increased can be used. Detection of the effect on the expression level *in vivo* based on the present invention may be conducted, for example, according to the following steps of:
(1) administering a candidate compound to a test animal; and
(2) measuring the expression level of the polynucleotide according to any one of above-described (a) through (d) in T cells of a test animal.

In an *in vitro* detection, for example, a method can be utilized, wherein a candidate compound is contacted with cells expressing polynucleotides according to any one of above-descried (a) through (d) to detect expression levels of these polynucleotides. More specifically, the method may be carried out according to the following steps of:
(1) contacting a candidate compound with cells that express the polynucleotide according to any one of above-described (a) through (d) ; and
(2) measuring the expression level of a polynucleotide according to any one of above-described (a) through (d).

In this invention, cells to be used in the step (1) can be obtained from an T-cell line, or by inserting these polynucleotides into an appropriate vector and then transfecting the vector to suitable host cells. Any vectors and host cells may be used so far as they are capable of expressing the polynucleotide of this invention. Examples of host cells in the host-vector system are *Escherichia coli* cells, yeast cells, insect cells, animal cells, and available vectors usable for each can be selected respectively.

Vectors may be transfected into the host by biological methods, physical methods, chemical methods, and so on. Examples of the biological methods include methods using virus vectors; methods using specific receptors; and the cell-fusion method (HVJ (Sendai virus) method, the polyethylene glycol (PEG) method, the electric cell fusion method, and microcell fusion method (chromosome transfer)). Examples of the physical methods include the microinjection method, the electroporation method, and the method using gene particle gun. The chemical methods are exemplified by the calcium phosphate precipitation method, the liposome method, the DEAE-dextran method, the protoplast method, the erythrocyte ghost method, the erythrocyte membrane ghost method, and the microcapsule method.

In the detection method of this invention, a T-cell line may be also used as the cell that expresses the polynucleotide according to any one of above-described (a) through (d). Examples of the T cell lines are the Molt-4 cell and Jurkat cell. In the screening method, first a candidate compound is contacted with the T-cell line. Then, in the T cell line, the expression level of the polynucleotide according to any one of above-described (a) through (d) is measured to select a compound that reduces the expression level of the polynucleotide compared to a control.

In the method of the present invention, expression levels of polynucleotides according to any one of above-described (a) through (d) can be compared by detecting the expression levels of not only proteins encoded by these polynucleotides but also the corresponding mRNAs. For carrying out the comparison of the expression level using mRNA, the step of preparing mRNA sample as described above is conducted in place of the step of preparing a protein sample. Detection of mRNA and protein can be carried out according to the known methods as described above.

Furthermore, based on the disclosure of this invention, it is possible to obtain the transcriptional regulatory region of the gene in the present invention and to construct a reporter assay system. Reporter assay system means an assay system of screening for a transcriptional regulatory factor that acts on the transcriptional regulatory region by using the expression level of a reporter gene that is located downstream of the transcriptional regulatory region and expressed under the control of the regulatory region as an index.

That is, this invention relates to a method of detecting the effect of a candidate compound on the activity of the transcriptional regulatory region of a gene having the nucleotide sequence of SEQ ID NO: 1, the method comprising the following steps of:
(1) contacting a candidate compound with a cell transduced with a vector containing the transcriptional regulatory sequence of a gene having the nucleotide sequence of SEQ ID NO: 1 and a reporter gene that is expressed under the control of this transcriptional regulatory sequence; and
(2) measuring the activity of the reporter gene.

A transcriptional regulatory region is exemplified by promoter, enhancer, as well as CAAT box, TATA box, and such, that are usually found in the promoter region. As a reporter gene, the CAT (chloramphenicol acetyltransferase) gene, the luciferase gene, growth hormone genes, and such can be utilized.

A transcriptional regulatory region of a gene of the present invention can be obtained as follows. Specifically, first, based on the nucleotide sequence of a cDNA disclosed in this invention, a human genomic DNA library, such as BAC library and YAC library, is screened by a method using PCR or hybridization to obtain a genomic DNA clone containing the sequence of the cDNA. Based on the sequence of the resulting genomic DNA, the transcriptional regulatory region of a cDNA disclosed in this invention is predicted and obtained. The obtained transcriptional regulatory region is cloned so as to be localized upstream of a reporter gene to prepare a reporter construct. The resulting reporter construct is introduced into a cultured cell strain to prepare a transformant for screening. By contacting a candidate compound with this transformant to detect the expression of a reporter gene, it is possible to assess the effect of the candidate compound on the transcriptional regulatory region.

Based on the method of detecting the effect on the expression level of the polynucleotides of this invention, it is possible to carry out screening for a compound that alters the expression level of the polynucleotides. This invention relates to a method of screening for a compound that alters the expression level of a polynucleotide according to any one of above-described (a) through (d), comprising following steps.

That is, the present invention relates to a method of screening for a compound that reduces the expression level of a polynucleotide of any one of above-described (a) through (d) , the method comprising the steps of detecting the effect of a candidate compound on the expression level of the polynucleotide *in vivo* and/or *in vitro,* and selecting a compound that reduces the expression level compared to a control.

Alternatively, this invention relates to a method of screening for a compound that. acts on the transcriptional regulatory region by the reporter assay utilizing the transcriptional regulatory region of the gene having the nucleotide sequence of SEQ ID NO: 1. Based on the results of reporter assay according to this invention, by selecting a compound that reduces the expression level of the reporter gene compared to a control, it is possible to obtain a compound that induces the expression of the gene having the nucleotide sequence of SEQ ID NO: 1.

Also, using the activity of the "B1153" protein of this invention as an index, it is possible to assess the effect of a test compound on the activity of the "B1153" protein of this invention. That is, this invention relates to a method of measuring the effect of a test compound on the activity of the "B1153" protein, the method comprising the following steps of:
(1) contacting a test compound with a protein encoded by the indicator gene; and
(2) measuring the activity of the protein.

As described in Examples, the present inventors observed the interaction of "B1153," the indicator protein in this invention, with the myosin-binding subunit 85, and skeletal muscle α2 actinin. Therefore, it is possible to assess the effect of a test compound on the activity of the "B1153" protein using its interaction with these molecules as an index. For the measurement of the intermolecular interaction, known methods can be used. One example of such assay methods is a pulldown assay.

Pulldown assay is carried out, for example, as follows. First, either one of the "B1153" indicator protein or its binding partner, myosin-binding subunit 85 (or skeletal muscle α2 actinin) is labeled by ³⁵S-methionine or such. The indicator protein and its binding partner used in the pulldown assay need not retain their complete molecular structures. For example, partial peptides containing domains necessary for the mutual binding may be used. As confirmed in Examples, the myosin-binding subunit 85 has binding activity to the B1153 protein at its C-terminal region, while the binding activity of the skeletal muscle α2 actinin to B1153 protein is confirmed at its middle section corresponding to 309 aa through 528 aa region.

Then, the indicator protein and its binding partner are incubated together with a test compound to collect the bound fraction. Indicator gene, if previously attached with a tag, facilitates the separation of the bound fraction. As a tag, the histidine tag and HA tag or such are used. By comparing the level of tag contained in the bound fraction to that in a control, the effect of the test compound is assessed.

Using the method of this invention for measuring the effect of a test compound on the activity of "B1153" protein, it is possible screen for a compound that has the regulatory function toward the activity of "B1153" protein. That is, this invention relates to a method of screening for a compound having the function to suppress the activity of "B1153" protein, the method comprising the following steps of:
(1) measuring the effect of a test compound on the activity of "B1153" protein; and
(2) selecting a compound that suppresses the activity of "B1153" protein compared to a control.

A compounds obtained as above suppresses the action of "B1153". Therefore, the indicator protein, whose expression is induced by T-cells, is inhibited by the administration of the compound, resulting in the control of an allergic disease.

Polynucleotide, antibody, cell line, animal model, "B1153" protein, binding partner for "B1153" protein, or such may be previously combined into a kit. Binding partner for the "B1153" protein means a component that interacts with the "B1153" protein. For example, the myosin binding subunit 85 and skeletal muscle α2 actinin were confirmed to interact with the "B1153" protein. These components, and compounds having partial structures thereof necessary for the interaction with B1153, can be used as the binding partner for the "B1153" protein in this invention.

More specifically, such a kit may be consisted of, for example, a cell that expresses an indicator gene, and a reagent for measuring the expression level of the indicator gene. As a reagent for measuring the expression level of an indicator gene, for example, an oligonucleotide that has at least 15 nucleotide sequence complementary to the polynucleotide comprising the nucleotide sequence of at least one indicator gene or to the complementary strand thereof is used. Alternatively, an antibody that recognizes a peptide comprising amino acid sequence of at least one indicator protein may be used as a reagent.

Alternatively, the "B1153" protein and its binding partner, myosin binding subunit 85 (or skeletal muscle α2 actinin) may be combined into a kit. The binding partner, myosin binding subunit 85 or skeletal muscle α2 actinin that composes a kit may be such fragments as described above containing the region necessary for their interaction with the "B1153" protein. Similarly, fragments of the "B1153" protein containing the region necessary for the interaction with the binding partner in a kit may be used. Such a kit can be used as the kit for assessing the effect of a test compound on the activity of the "B1153" protein. Furthermore, the "B1153" protein or its binding partner may be provided in a state where they are immobilized onto microbeads or reaction vessel. Kits in which components necessary for the reaction are immobilized are useful for the high throughput screening.

In these kits may be packaged a substrate compound used for the detection of the indicator, medium and vessel for cell culturing, positive and negative standard samples, and furthermore, a manual describing how to use the kit. A kit of this invention for detecting the effect of a candidate compound on the expression level of the "B1153" gene can be used as a kit for screening for a compound that modifies the expression level of the "B1153" gene.

Test candidate compounds used in these methods include, in addition to compound preparations synthesized by existing chemical methods such as steroid derivatives and compound preparations synthesized by combinatorial chemistry, mixtures of multiple compounds such as extracts from animal or plant tissues, or microbial cultures and their purified preparations, and so on.

Compounds selected by the screening method of this invention are useful as the therapeutic agent for an allergic disease. Alternatively, an antisense DNA that can suppress the expression of the indicator gene, and furthermore, antibody recognizing the protein encoded by the indicator gene are also useful as the therapeutic agent for an allergic disease. A therapeutic agent for allergic disease of the present invention can be formulated by including a compound selected by the screening methods as the effective ingredient, and mixing with a physiologically acceptable carrier, excipient, diluent, and such. Aiming at the amelioration of allergic symptoms, the therapeutic agent for allergic disease of this invention can be administered orally or parenterally.

Oral drugs can take any dosage forms selected from the group of granule, powder, tablet, capsule, solution, emulsion, suspension, and so on. Injections can include subcutaneous injection, intramuscular injection, intraperitoneal injection, and so on.

Furthermore, for administering a compound that is composed of protein, a therapeutic effect can be achieved by introducing a gene encoding the protein into the living body using gene therapeutic techniques. The techniques for treating disease by introducing a gene encoding a therapeutically effective protein into the living body and expressing it therein are known in the art.

Alternatively, an antisense DNA can be incorporated downstream of an appropriate promoter sequence to be administered as an antisense RNA expression vector. When this expression vector is introduced into T cells of an allergic disease patient, the therapeutic effect on allergic disease is achieved by the reduction of the expression level of the gene through the expression of the corresponding antisense gene. For introducing the expression vector into T cells, methods performed either *in vivo* or *ex vivo* are known.

Although the dosage may vary depending on the age, sex, body weight, and symptoms of a patient; treatment effects; method for administration; treatment duration; type of active ingredient contained in the drug composition; and such, a range of 0.1 to 500 mg, preferably 0.5 to 20 mg per dose for an adult can be administered. However, the dose changes according to various conditions, and thus in some case a more smaller amount than that mentioned above is sufficient whereas an amount above the above-mentioned range is required in other cases.

### Brief Description of the Drawings

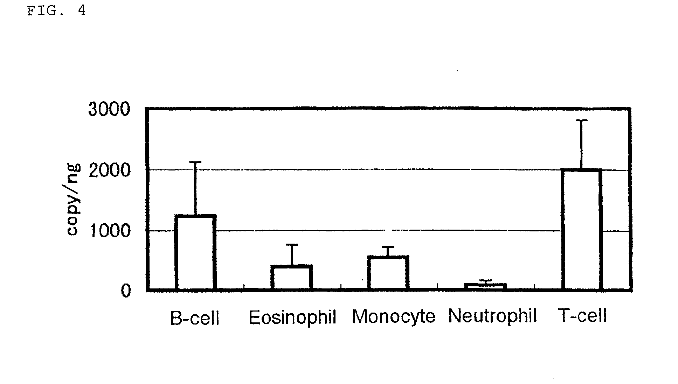

Fig. 1 is a diagram showing the spatial relationships among the approximately 2.0-kb sequence (SEQ ID NO: 8) obtained on the genomic sequence (AC002453) by the 5' RACE method, exons predicted by GENSCAN and Gene Finder, and primers set up based on these predictions. A box shown in the vicinity 10.7 kb to 12.7 kb refers to the DD fragment and the extended region obtained by the 5' RACE method, represented by "5'-RACE" and "DD", respectively. Solid and open boxes represent exons predicted by GENSCAN and Gene Finder, respectively. Arrows indicate primers and their 3'-end directions.
Fig. 2 is a graph showing the distribution of the "B1153" expression level in the case where subjects were divided into groups of the normal healthy people and patients (those suffering from atopic dermatitis).
Fig. 3 is a series of photographs showing the results of Northern hybridization for measuring the "B1153" expression level in a variety of tissues or cells. Numerals in the figure correspond to respective tissues as follows:
   I:
      i heart
      ii brain
      iii placenta
      iv lung
      v liver
      vi skeletal muscle
      vii kidney
      viii pancreas
   II:
      i spleen
      ii thymus
      iii prostate
      iv testis
      v ovary
      vi small intestine
      vii large intestine
      viii peripheral blood leukocyte
   III:
      i cerebellum
      ii cerebral cortex
      iii medulla
      iv spinal cord
      v occipital pole
      vi frontal lobe
      vii temporal lobe
      viii putamen
   IV:
      i tonsil
      ii caudatum
      iii corpus callosum
      iv hippocampus
      v whole brain
      vi nigra
      vii thalamus
   V:
      i spleen
      ii lymph node
      iii thymus
      iv peripheral blood leukocyte
      v bone marrow
      vi fetal liver
   VI:
      i promyelocytic leukemia HL-60
      ii HeLa cell S3
      iii chronic myelocytic leukemia K-562
      iv lymphoblastic leukemia MOLT-4
      v Burkitt's lymphoma Raji
      vi colorectal adenocarcinoma SW480
      vii lung carcinoma A549
      viii melanoma G361
Fig. 4 is a graph showing the results of quantitative PCR for measuring the "B1153" gene expression level in various immunocytes. In this figure, the ordinate represents the "B1153" gene expression level (copy/ng) corrected for the β actin, and the abscissa shows the cell type.
Fig. 5 is a diagram summarizing the relationship of the "B1153" gene of the present invention with the known nucleotide sequences: KIAA1861 (Accession No. AB058764), FLJ23581 (Accession No. AK027234), and FLJ20097.

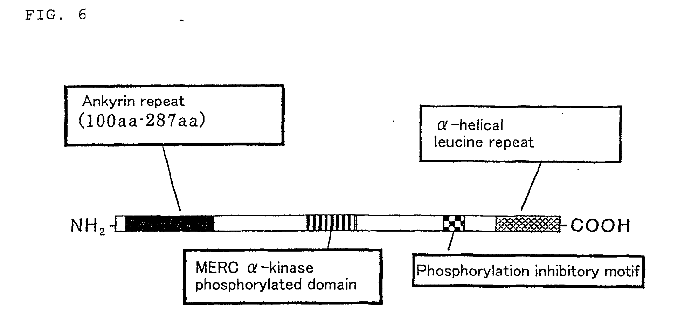

Fig. 6 is a diagram showing the structure of the myosin binding subunit 85 (Accession No. AF312028) whose interaction with the "B1153" protein was detected in Example 12.

### Best Mode for Carrying out the Invention

The present invention will be explained in more detail below with reference to examples, but is not to be construed as being limited thereto.

### [Example 1] Collection of blood samples from patients and normal healthy subjects

To isolate genes that show different expression in an allergic disease-specific manner, blood samples were collected from patients and normal healthy volunteers selected after analysis of their symptoms. The blood samples were collected as a group of normal healthy subjects and patients with a very mild case of allergy, a group of patients with bronchial asthma, and a group of patients with atopic dermatitis from 12, 23 and 24 people, respectively. The amount of mite-antigen-specific IgE was measured using a part of the blood samples.

The specific IgE measurement was conducted according to the CAP radioallergosorbent test (CAP RAST) method, a modified RAST method, that uses a paper disk as a solid phase. Serum from Pharmacia, which has a standard antibody titer, was used as the standard to determine IgE antibody titers in respective samples. The obtained values were scored.

Scores of mite-specific IgE antibody titers of each subject are shown in the column under the title of "IgE score" of Table 1. As shown in the table, the scores in the group of normal healthy subjects and patients of very mild case of allergy were "0" except for one patient of a very mild case. On the other hand, the patient group showed high scores, indicating that patients in this group are allergic toward the mite antigen.

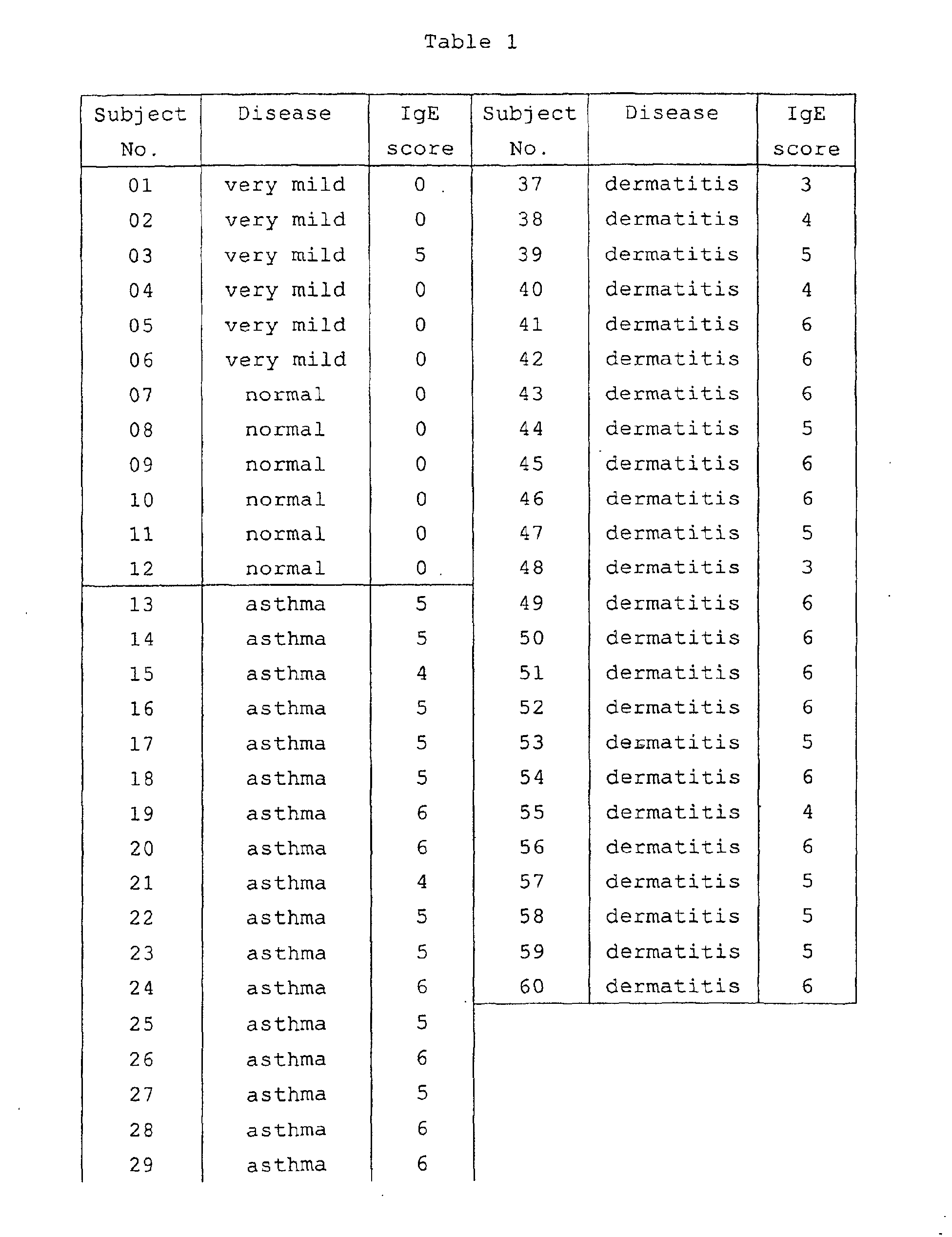

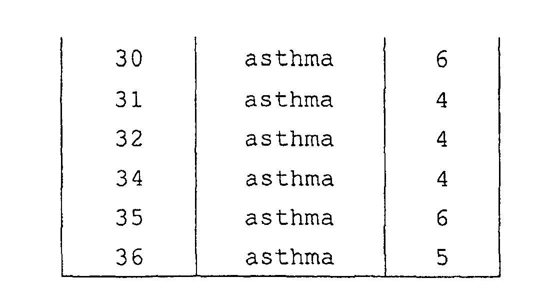

### [Example 2] Preparation of lymphocyte fractions from blood samples

T-cells were prepared from 10 ml blood sample as follows. First, 1 ml heparin (purchased from Novo Co., etc.) was thoroughly spread over the 10 ml-syringe wall surface, and then 10 ml blood sample including a final concentration of 50 units/ml heparin was collected. For blood collection, two 22G needles for each person were prepared. After removing the needle from the syringe, the blood sample was transferred to a 50-ml centrifuge tube (polypropylene) . The tube was centrifuged at 1500 rpm for 5 min at room temperature and then 1.1 ml was taken from as close to the surface as possible. After further 15000 rpm centrifugation for 5 min at 4°C, 1 ml of the supernatant was collected as plasma. An equal amount (9 ml) of 0.9% NaCl containing 3% dextran (Nacalai) was added to the remaining sample. This mixture was inverted gently several times, and then was left standing for 30 min at room temperature. PRP (platelet rich plasma) was transferred to a new 15 ml centrifuge tube and centrifuged at 1200 rpm (equivalent to 150x g for the Tomy centrifuge) for 5 min at room temperature. After the centrifugation, platelets were present in the supernatant. Precipitated cells were resuspended in 5 ml Ca and Mg-free HBSS (GIBCO, etc.). The cell suspension was layered on the top of a 5 ml Ficoll Paque (Pharmacia) -containing Falcon tube (2006 or 2059, polypropylene) with a capillary pipette. After centrifuging the tube at 1200 rpm for 5 min, it was further centrifuged at 1500 rpm (equivalent to 400x g for the Tomy centrifuge) for 30 min at room temperature. As a result, granulocytes and erythrocytes were precipitated, and lymphocytes, monocytes, and platelets were included in the middle layer, with the Ficoll layer between the precipitate and the middle layer.

The middle layer was collected using a Pasteur pipette. Two to three volumes of bovine serum albumin (BSA)/phosphate buffered saline (PBS) (0.5% BSA, 2 mM EDTA in PBS, pH 7.2, degassed just before use) were added thereto, and the mixture was centrifuged at 1200 rpm for 5 min at 4°C. The precipitate was collected and washed twice with BSA/PBS solution. After the second wash, cells were resuspended in 5 ml BSA/PBS, and a portion of the supernatant was diluted two-fold with trypan blue to count the cell number. Total cell numbers were about 1x 10⁷, and the suspension was used as lymphocyte fraction.

### [Example 3] T-cell separation from lymphocyte fraction

The lymphocyte fraction obtained in Example 2 was centrifuged at 1200 rpm for 5 min at 4°C, and the precipitate was resuspended in BSA/PBS at 10⁸ cells/100 µl. The volume was approximately 20 µl. The cell suspension was transferred to an Eppendorf tube (1.5 ml), and then CD3 microbead solution was added thereto. This sample was allowed to stand at 4 to 10°C for 30 min (not on ice) and was further treated using magnetic cell sorter (MACS, Miltenyi Biotech Inc.) by the following procedure.

An MS⁺/RS⁺ column was set on Mini MACS or Vario MACS separation unit (without needles). 500 µl of BSA/PBS was gently applied onto the column, and the buffer was run off. Then CD3 microbead-labeled cells were applied onto the column. The column was washed three times with 500 µl BSA/PBS (B-cell fraction) . The column was detached from the separation unit and set onto a tube to collect the eluate. 1 ml of BSA/PBS was applied onto the column, and CD3-positive cells were eluted rapidly using a plunger attached to the column. The eluate was used as T-cell fraction.

The obtained T-cell fraction was centrifuged at 1200 rpm at 4°C for 5 min. The precipitate was washed twice with BSA/PBS. After the second wash, the cells were resuspended in 1 ml BSA/PBS, and a portion of the suspension was diluted two-fold with trypan blue to count the cell number. Total cell numbers were approximately 4x 10⁶.

### [Example 4] Total RNA preparation from T-cells

Total RNA was prepared from T-cells using RNeasy Mini (Qiagen) basically following the manufacturers' instruction. All manipulations were carried out at room temperature, wearing gloves. Four-fold volume of ethanol was added to the wash buffer RPE. To the lysis buffer RLT, 10 µl/ml of 2-mercaptoethanol was added.

The cell suspension was centrifuged at 1000 to 1200 rpm for 5 min, and the supernatant was removed by aspiration. The precipitate was resuspended in 350 µl lysis buffer RLT (containing 2-mercaptoethanol) . At this step, the cell lysate in the lysis buffer RLT could be stored at -70°C. The frozen stored cell lysate was thawen by incubation at 37°C for 10 to 15 min, and, if insoluble matter was observed, was centrifuged for 3 min at maximum speed to collect the supernatant alone. The lysate was homogenized by syringe with a 20G Cathelin needle, and then 350 µl lysate was applied onto QIA shredder with a Pipetman, and centrifuged at 1500 rpm for 2 min to collect the eluate. 350 µl of 70% ethanol was added thereto and mixed well by pipetting.

An RNeasy spin column was fixed to the attached 2-ml tube, and the lysate mixture was applied onto the column. The column was centrifuged at 8000x g (11500 rpm) for 1 min, and the flow through was discarded. Then 700 µl wash buffer RW1 was applied onto the column, and the column was left standing capped for 5 min. The column was centrifuged at 11500 rpm for 15 seconds, and the flow through was discarded. The column was attached to a new 2-ml tube, 500 µl wash buffer RW1 was applied onto the column, centrifuged at 11500 rpm for 15 min, and the flow through was discarded. 500 µl wash buffer RPE was applied onto the column, and centrifuged at full speed for 2 min. The column was attached to a new tube (1.5 ml), 30 µl of DEPC treated water was applied thereto, and the capped column was allowed to stand for 10 min. The column was centrifuged at 11500 rpm for 10 min to obtain total RNA. The concentration of the RNA was measured. If the amount was low, the column was set again onto a new 1.5-ml tube, and 30 µl of DEPC treated water was applied thereto. Then the column was left standing capped for 10 min, and centrifuged at 11500 rpm for 10 min to obtain total RNA.

### [Example 5] DNase treatment of total RNA

In order to remove DNA from the total RNA prepared from the T-cells, DNase treatment was performed. The treatment was conducted in a reaction mixture containing 2 units of DNase (Nippon Gene) and 50 units of RNase inhibitor (Pharmacia) in 100 µl of 1x DNase buffer (Nippon Gene) . After incubating this mixture at 37°C for 15 min, an equal volume of PCI (phenol: chloroform: isoamyl alcohol = 25:24:1) was added thereto, and the tube was vortexed. The tube was centrifuged at 12000 rpm at room temperature for 10 min, and the upper phase (aqueous phase) was transferred to a new 1.5-ml tube. One tenth volume of 3 M sodium acetate (pH 5.2) and 2.5 volumes of 100% ethanol with 1 µl of Ethachinmate were added thereto, and the mixture was inverted several times. After allowing the tube to stand at -20°C for 15 min, it was centrifuged at 12000 rpm for 15 min at 4°C. The supernatant was removed, and 70% ethanol was added to the precipitate. After tapping the tube until the precipitate was detached from the tube, the supernatant was completely removed. The precipitate was dried for 3 min and dissolved in 10 to 20 µl DDW (DNase and RNase free). The concentration was measured, and the sample was stored at -80°C until use.

### [Example 6] Differential Display (DD) analysis using total RNA prepared from T-cells

Fluorescent Differential Display (abbreviated to DD) analysis using total RNA prepared from T-cells was carried out according to the literature (T. Ito et al., 1994, FEBS Lett. 351: 231-236). The total RNA prepared from the T-cells was reverse transcribed to obtain cDNA. In the first DD-PCR reaction, 0.2 µg each of total RNA was used for three types of anchor primers to synthesize cDNAs. In the second DD-PCR reaction, 0.4 µg each of total RNA was used for the synthesis of cDNAs using three types of anchor primers, respectively. In both cases, the cDNAs were diluted to a final concentration equivalent to 0.4 ng/µl RNA and used for further experiments. The DD-PCR reaction was carried out using an amount of cDNA equivalent to 1 ng RNA per reaction. The reaction mixture composition was as shown in Table 2.

**Table 2**

| | |
|---|---|
| cDNA (equivalent to 0.4 ng/µl RNA) | 2.5 µl |
| Arbitrary primer (2 µM) | 2.5 µl |
| 10x AmpliTaq PCR buffer | 1.0 µl |
| 2.5 mM dNTP | 0.8 µl |
| 50 µM anchor primer | 0.1 µl |
| (GT15A, GT15C, or GT15G) | |
| Gene Taq (5 U/µl) | 0.05 µl |
| AmpliTaq (5 U/µl) | 0.05 µl |
| dH₂O | 3.0 µl |
| Total volume | 10.0 µl |

The PCR reaction was carried out at following condition: 1 cycle of "95°C for 3 min, 40°C for 5 min, and 72°C for 5 min"; subsequently 30 cycles of "94°C for 15 sec, 40°C for 2 min, and 72°C for 1 min"; after these cycles, 72°C for 5 min; and then continuously 4°C.

Reactions were conducted using 287 primer pairs: i.e., anchor primers GT15A (SEQ ID NO: 3), GT15C (SEQ ID NO: 4), and GT15G (SEQ ID NO: 5) were used in combination with arbitrary primers AG 1 to AG 110, AG 111 to AG 199, and AG 200 to AG 287, respectively. As for the arbitrary primers, oligomers having 10 nucleotides with a GC content of 50% were designed and synthesized.

For gel electrophoresis, a 6% denaturing polyacrylamide gel was prepared, and 2.5 µl sample from the PCR was applied and run under 40 W for 210 min. After electrophoresis, the gel was scanned by Hitachi fluorescence imaging analyzer FMBIO II, and the gel image was obtained by detecting fluorescence.

DD analysis was carried out twice. Reproducible bands that differed in the expression level between the patient and normal healthy groups were excised from the gels, and sequencing was performed. As a result, one gene (DD analysis band ID B1153-03; hereafter referred to as the "B1153" gene) that differed in the expression level between the patient and normal healthy groups was identified. The primer set used for amplifying the band ID B1153-03 is shown below. In addition, the nucleotide sequence of DD band of B1153-03 is set forth in SEQ ID NO: 6.
Band ID: B1153-03
Fragment length: 184 bp (excluding the nucleotide sequence of the primer)
Anchor primer: GT15A
Name of arbitrary primer: AG00103
Sequence of arbitrary primer: TGACCTGAGT (SEQ ID NO: 7)

### [Example 7] Elongation of nucleotide sequence

### (1) Nucleotide sequence analysis by 5' RACE

Based on the nucleotide sequence determined in Example 6, the nucleotide sequence analysis of the gene of this invention was conducted using the 5' RACE method. According to the protocol of Marathon cDNA Amplification Kit (CLONTECH) , PCR was carried out using a Human Leukocyte Marathon Ready cDNA (CLONTECH) as a template, AP1 primer attached to the kit, and "B1153"-specific 1153-2R primer. Furthermore, PCR was conducted using this amplified fragment as a template, "AP2", which is a sequence within the adapter, and the 1153-2R primer. As a result of subcloning the amplified fragment followed by sequencing, an approximately 2.0 kb sequence (SEQ ID NO: 8) comprising the "B1153" sequence was obtained.

### Primer sequence

1153-2R: AACCTCTACTCAACAACTCACCCCATAA (SEQ ID NO: 9)

### (2) Analysis of genomic sequence

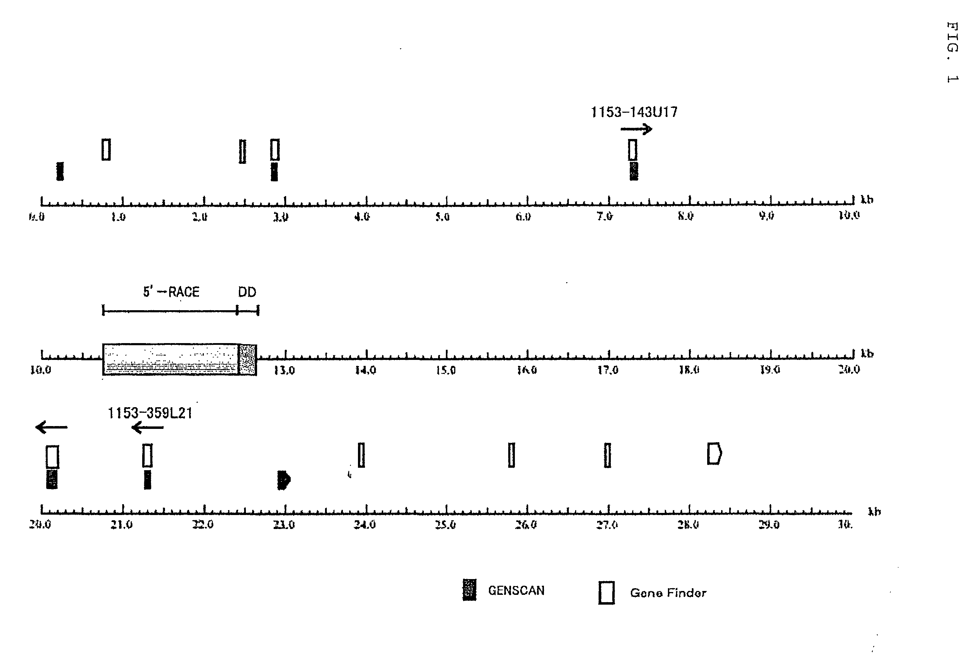

Multiple genomic sequences showed homology to the 2.0-kb sequence obtained in (1) above. Among them, for the genomic sequence (AC002453) that showed the highest homology, the exon prediction was carried out using an exon searching software (GENSCAN, GRAIL, Gene Finder or ER). As a result, a putative gene was found. The 2.0 kb sequence was found to be in the intron region of that putative gene. Based on the hypothetical exon sequence near the 2.0 kb sequence, a plurality of primers were designed. Positional relationship among exons predicted by GENSCAN and Gene Finder as well as those primers designed based on these predictions were shown in Fig. 1.

From the total RNA prepared from the human peripheral blood-derived T-cells and PBMC (peripheral blood mononuclear cell) as well as the human peripheral blood leukocyte-derived poly (A) RNA, cDNA was synthesized to be used as a template for PCR. With this template, PCR was conducted using two sets or more of the above-described primers. The PCR-amplified fragment was cloned and then sequenced to confirm whether it was the hypothetical exon or not. As a result, by PCR using 1153-143U17 and 1153-359L21 as the forward and reverse primers respectively, the actual existence of the predicted 199 bp gene sequence (Sequence A, SEQ ID NO: 10) was confirmed.

### Primer sequences

1153-143U17: GAAAAGCCCTCAAGAAA (SEQ ID NO: 11), and

1153-359L21: TTGTCTCTATACGCCTCTAAT (SEQ ID NO: 12).

### (3) Nucleotide sequence analysis by EST clustering

From the public databases, the EST sequence (GenBank Accession#: AI793062, 524 bp) that is identical to Sequence A, and EST sequences that were observed to be homologous to Sequence A were found. These ESTs were clustered to obtain a 681 bp nucleotide sequence. In this sequence, the primers 1153EST-F (5'-GGGTCATTTGTGTAGTGGCTCGG-3'; SEQ ID NO: 13) and 1153EST-R (5'-CCTCCTCCAGCATTTCACTTAACCG-3'; SEQ ID NO: 14) were designed. Using these primers, PCR was carried out with the same library as that in (2) as a template to obtain a 649 bp PCR amplified fragment. As a result of sequencing of this PCR-amplified fragment, it was found to be identical to the sequence obtained by EST clustering.

### (4) Nucleotide sequence analysis by clustering

Of the 649 bp nucleotide sequence thus determined, based on the 601 bp nucleotide sequence excluding that derived from the primer, the exon analysis on the genomic sequence (AC002453) was further conducted. The following pair of PCR primers were designed based on the hypothetical exon sequences predicted downstream towards the 3'-end, cloning and sequencing of the PCR product were carried out by the same procedure as that in (2).

### Primers

1153-3'-207U18: 5'-GAGAACAGCCAAGTGACC-3' (SEQ ID NO: 15) and

1153-3'-896L21: 5'-TTTTTCCAAGAAGTCGATAAG-3' (SEQ ID NO: 16).

As a result of this PCR amplification, a 636-bp gene fragment was amplified, the sequence of which was found to contain the predicted exon sequence. The 636-bp nucleotide sequence amplified using the above-described primers is set forth in SEQ ID NO: 17. By assembling the here obtained 636-bp sequence and the previously obtained 601-bp sequence, the 824-bp nucleotide sequence as a whole (SEQ ID NO: 25) was obtained, which contained a region (nucleotides 50 to 820) that is seemingly a portion of ORF on the 3'-side, encoding 257 amino acids (SEQ ID NO: 26). No existing sequence that shows a homology to this amino acid sequence was found out, so that "B1153" was considered to encode a novel protein.

### [Example 8] Quantification of expression level using ABI-7700

The total RNA was prepared from T cells collected from 10 each of normal healthy subjects and patients with light, moderate, and severe atopic dermatitis, all of them being different from those in Example 1. Parts of the total RNA samples were used for quantifying the gene expression level by TaqMan method with ABI-PRISM 7700. This TaqMan method is a system for detecting and quantifying PCR-amplified DNA strands in real-time using fluorescence dyes.
In this method, a primer set prepared based on the "B1153" nucleotide sequence determined based on the sequences of DD bands in Example 7 was used. Furthermore, the TaqMan probe was used after labeled with FAM (6-carboxy-fluorescein) and TAMRA (6-carboxy-N,N,N',N'-tetramethylrhodamine) at 5'-end and 3'-end, respectively. Nucleotide sequences of the used primer sets and TaqMan probe are shown below.

1153-F2: AAAGCCCTCAAGAAAGCCTCA (SEQ ID NO: 18),

1153-R2: GGTCACTTGGCTGTTCTCGAA (SEQ ID NO: 19), and

TaqMan probe: TGATCTTGGTGCCATAGAGAGTCTCCGG (SEQ ID NO: 20).

cDNA was used as a template which was prepared by reverse transcription from the total RNA using poly-T (12 to 18 mer) as primers. In order to make a standard curve for the calculation of copy numbers, a plasmid clone containing the nucleotide sequence amplified using both primers was prepared, and serial dilutions thereof were utilized as the template for the reaction. The reaction mixture composition for monitoring PCR amplification is shown in Table 3.

**Table 3**

| Reaction mixture composition for ABI-PRISM 7700 (amount per well) | |
|---|---|
| Sterile distilled water | 25.66 (µl) |
| 10x TaqMan buffer A | 5 |
| 25 mM MgCl₂ | 7 |
| dATP (10 mM) | 1.2 |
| dCTP (10 mM) | 1.2 |
| dGTP (10 mM) | 1.2 |
| dUTP (10 mM) | 1.2 |
| Forward Primer (100 µM) | 0.15 |
| Reverse Primer (100 µM) | 0.15 |
| TaqMan Probe (6.7 µM) | 1.49 |
| AmpliTaq Gold (5 U/µl) | 0.25 |
| AmpErase UNG (1 U/µl) | 0.5 |
| Template solution | 5 |
| Total volume | 50 |

In order to correct the difference in the cDNA concentrations between the samples, the same quantitative analysis was carried out for the β-actin gene that was used as internal standard, and, by performing correction based on its copy number, the copy number of the target gene was calculated. For the quantification of the β-actin gene, the human cDNA was used as a template.

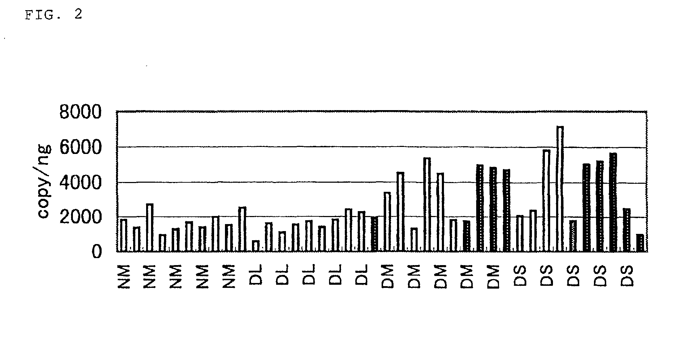

As the primers and probe for the measurement of β-actin were used those attached to TaqMan β-actin Control Reagents (PE Biosystems). Their nucleotide sequences are as shown below. The "B1153" gene expression levels (copy/ng RNA) corrected for that of β-actin are shown in Table 4 and Fig. 2. 
β-Actin forward primer (SEQ ID NO: 21)
TCA CCC ACA CTG TGC CCA TCT ACG A
 
β-Actin reverse primer (SEQ ID NO: 22)
CAG CGG AAC CGC TCA TTG CCA ATG G
 β-actin TaqMan probe (SEQ ID NO: 23) 5'-(FAM)ATGCCC-T(TAMRA)-CCCCCATGCCATCCTGCGTp-3'
FAM: 6-carboxy-fluorescein
TAMRA: 6-carboxy-N,N,N',N'-tetramethylrhodamine

Using the obtained data in Table 4, a statistical analysis of significant variance in the gene expression level among each group was conducted by the parametric technique. The assay between two groups, namely the normal healthy group and patient group (including patients with the light, moderate and, severe atopic dermatitis), was conducted by the t-test. Subjects were also divided into four groups: the normal healthy group (NM) as well as the three atopic dermatitis patient groups of light (DL), moderate (DM) and severe (DS) cases, respectively. The test between two groups among them was conducted by the Tukey's multiple comparison test, using a SAS Preclinical Package Version 4.0 of The SAS SYSTEM (SAS Institute, Inc.). The results of the t-test and Tukey's multiple comparison test are shown in Table 5.

**Table 4**

| Symptom | | copy/ng |
|---|---|---|
| Normal healthy subject | | |
| 1 | NM | 1816.47 |
| 2 | NM | 1368.00 |
| 3 | NM | 2726.58 |
| 4 | NM | 949.69 |
| 5 | NM | 1282.75 |
| 6 | NM | 1670.67 |
| 7 | NM | 1397.46 |
| 8 | NM | 1985.45 |
| 9 | NM | 1505.48 |
| 10 | NM | 2538.33 |

| Light atopic dermatitis | | |
|---|---|---|
| 11 | DL | 584.77 |
| 12 | DL | 1620.12 |
| 13 | DL | 1095.80 |
| 14 | DL | 1552. 95 |
| 15 | DL | 1724.20 |
| 16 | DL | 1395.51 |
| 17 | DL | 1824.83 |
| 18 | DL | 2435.67 |
| 19 | DL | 2257.11 |
| 20 | DL | 1919.66 |

| Moderate atopic dermatitis | | |
|---|---|---|
| 21 | DM | 3385.60 |
| 22 | DM | 4507.43 |
| 23 | DM | 1296.35 |
| 24 | DM | 5346.69 |
| 25 | DM | 4490.46 |
| 26 | DM | 1808.76 |
| 27 | DM | 1703.40 |
| 28 | DM | 4977.20 |
| 29 | DM | 4815.31 |
| 30 | DM | 4723.18 |

| Severe atopic dermatitis | | |
|---|---|---|
| 31 | DS | 2050.58 |
| 32 | DS | 2391.61 |
| 33 | DS | 5799.96 |
| 34 | DS | 7142.03 |
| 35 | DS | 1754.79 |
| 36 | DS | 5032.68 |
| 37 | DS | 5208.56 |
| 38 | DS | 5662.86 |
| 39 | DS | 2495.72 |
| 40 | DS | 984.57 |

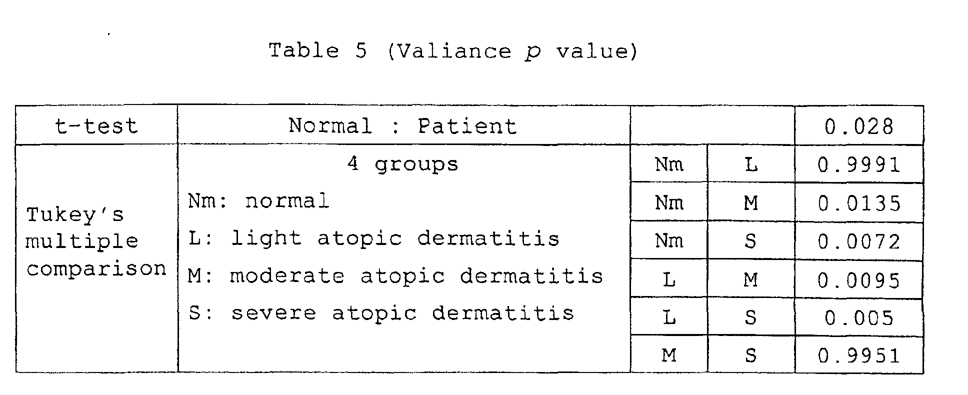

As a result of statistical analysis, it was confirmed by the t-test that the B1153 gene expression level was significantly high in the atopic dermatitis patient group compared to the normal healthy group (p = 0.028). From the results of Tukey's multiple comparison test, it was confirmed that the B1153 gene expression level was significantly higher in the moderate or severe atopic dermatitis patient group compared to the normal healthy group or light atopic dermatitis patient group. Variance *p* values were *p* = 0.0135 between the normal and moderate patient groups, *p* = 0.0072 between the normal and severe patient groups, and *p* = 0.0095 between the light and moderate patient groups, and *p* = 0.005 between the light and severe patient groups, respectively. The above-described information indicates that the "B1153" gene expression is high in atopic dermatitis patients. These results indicate that measurement of this gene expression is valuable for diagnosing atopic dermatitis. From the viewpoint of medical treatment, this gene derived from T-cells is useful as the treatment target or diagnostic marker for atopic dermatitis.

### [Example 9] Measurement of "B1153" expression level in various tissues and cells

Using membrane filters to which mRNAs prepared from human various tissues and cancer cell lines were transferred, the "B1153" expression level was examined. In this case, the following six types of filters (all from CLONTECH) were used:
Human MTN Blot,
Human MTN Blot II,
Human Brain II,
Human Brain IV,
Human Immune System MTN Blot II, and
Human Cancer Cell Line MTN Blot.

The 649 bp DNA fragment amplified by PCR using the "B1153" primers 1153EST-F and 1153EST-R that were employed in examples (7) to (3) was labeled with ³²P using a Random Primer Labeling Kit (TAKARA) to be used as a probe. Using an Express Hybridization Solution (CLONTECH) , Northern hybridization and membrane washing were carried out according to the attached manual. The washed membrane was exposed to an imaging plate to develop the images using a Molecular Imager System (BIO-RAD).

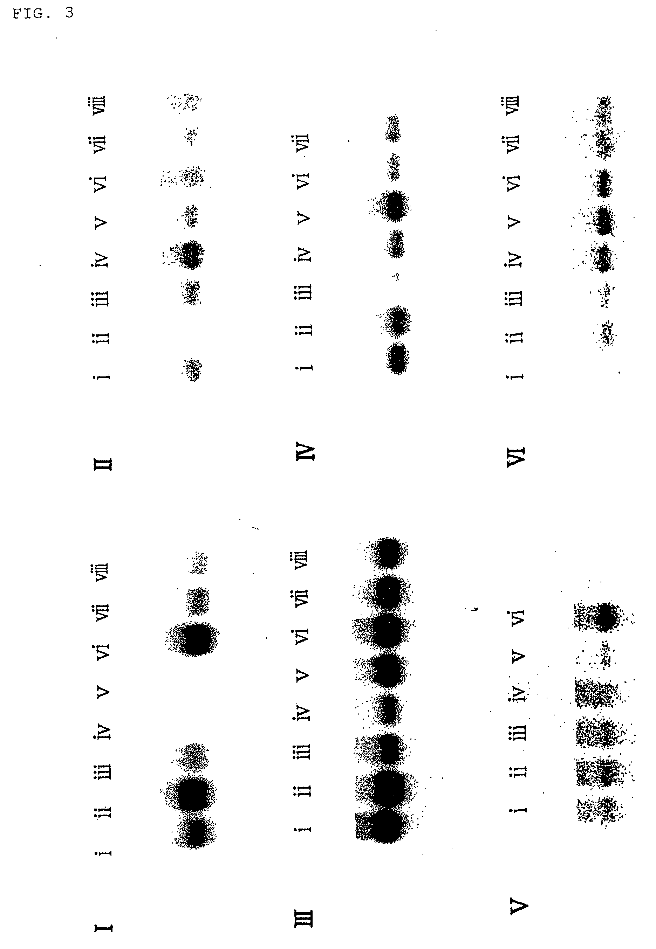

The results are shown in Fig. 3. In almost all the tissues including immune-related tissues and cancer cell lines, the approximately 4 kb mRNA was detected. A strong expression of the "B1153" gene according to this invention was observed in the brain in particular.

### [Example 10] Expression of "B1153" gene in various immunocytes

"B1153" gene expression was examined in cells separated from peripheral blood collected from five normal healthy subjects. Separation of T-cells (T) was carried out as follows. To the whole blood collected was added 3% dextran solution, and the mixture was allowed to stand at room temperature for 30 min to sediment erythrocytes. The leukocyte fraction in the upper layer was collected, layered on a Ficoll solution (Ficoll-Paque PLUS; Amersham Pharmacia Biotech), and centrifuged at 1500 rpm for 30 min at room temperature. The granulocyte fraction recovered in the lower layer was reacted with the CD16 antibody magnetic beads at 4°C for 30 min, and cells that were not trapped and eluted in the separation step using MACS were used as the eosinophils in experiments. After the elution of eosinophils, neutrophils (N) were prepared by releasing the cells, which were trapped with CD16 antibody magnetic beads, from the magnetic field, eluting, and recovering. On the other hand, the monocyte fraction recovered in the middle layer by the Ficoll-centrifugation was separated into the fraction eluted from MACS CD3 antibody magnetic beads (mixture of M (monocyte) and B cell) and fraction trapped therein (T-cell fraction). Then, using MACS CD14 antibody magnetic beads, the eluted fraction was separated into the eluted fraction (B cell fraction) and trapped fraction (monocyte fraction), and those three fractions were referred to as the purified T cells, B cells, and monocytes.

Eosinophils were solubilized using Isogen, while neutrophils, T cells, B cells and monocytes were solubilized with RNeasy (Qiagen), and total RNA were extracted, treated with DNase (by the same methods as described above), and subjected to the gene expression analysis. Primers, probes, and so forth used were the same as above. Average expression levels (AVERAGE: copy/ng (corrected value)) in these blood cells are shown below together with SDs. Furthermore, the measurement results were summarized in Fig. 4. From these results, it was revealed that the gene expression is highest in T-cells.

| | | | |
|---|---|---|---|
| Eosinophil (E) | 394.15±358.37 | T-cell (T) | 2004.11±803.91 |
| Neutrophil (N) | 85.75±73.90 | Monocyte (M) | 537.70±166.95 |
| B-cell (B) | 1231.60±892.66 | | |

### [Example 11] Isolation of cDNA clone

Using a GENE TRAPPER II system (INVITROGEN), a cDNA clone of the "B1153" gene was isolated. Then, using the 284-bp (SEQ ID NO: 24) B1153 probe that corresponds to 64 bp through 347 bp in SEQ ID NO: 25, screening of the GT II Full-length Testis cDNA library (INVITROGEN) for homology to this clone was performed. As a result, a 3596 bp full-length cDNA clone containing ORF was obtained. The determined cDNA nucleotide sequence and amino acid sequence of a protein encoded by this nucleotide sequence are set forth in SEQ ID NOs; 1 and 2, respectively.

A search of databases for homology of this sequence information revealed that the nucleotide sequence from 505 bp to 2148 bp and that from 1420 bp to 3596 bp in the "B1153" gene nucleotide sequence of SEQ ID NO: 1 were highly homologous to KIAA1861 (Accession No. AB058764) and FLJ23581 (Accession No. AK027234), respectively. However, the sequence from 1 bp to 504 bp was a novel sequence. Relationships among these nucleotide sequences are summarized in Fig. 5.

### [Example 12] Screening for interacting proteins by yeast two-hybrid system

By the PROQUEST™ TWO-Hybrid system (Invitrogen) utilizing the *in vivo* gene expression system of GAL4 series yeast, a search for a protein that interacts with the protein of this invention was carried out. Screening for a protein that interacts with B1153 was conducted for the Human Brain cDNA library with the full-length ORF region (86 bp to 2977 bp) of the B1153 gene as a bait. In this case, protocol according to the manual attached to the system was used.

As a result, five positive clones that interact with the B1153 protein were obtained. Among them, four clones were partial sequences of the myosin binding subunit 85 (Accession No. AF312028) (Fig. 6) , and one clone was a partial sequence of the skeletal muscle alpha 2 actinin (Accession No. M86406). Sequence ranges that the obtained prey clones retain are shown below. Herein, information in brackets represent GenBank Accession Nos. Based on these results, it is possible to identify the region necessary for the interaction with B1153 within the amino acid sequences composing the myosin binding subunit 85 or skeletal muscle alpha 2 actinin.

For example, as for the myosin binding subunit 85, since up to three out of the four prey vectors shown below contain the C-terminus region following the 590 aa, the C-terminus region of the myosin binding subunit 85 is considered to interact with B1153. Similarly, as for the skeletal muscle alpha 2 actinin, since the prey vector containing its middle region corresponding to 309 aa through 528 aa was positive, the middle section of the skeletal muscle alpha 2 actinin corresponding to 309 aa through 528 aa is considered to interact with B1153.

Full-length 782 amino acids of myosin binding subunit 85 (No. AF312028)
Prey-No. 3: 1784 bp to 2541 bp (590 aa to 782 aa and 3'-UTR),
Prey-No. 4: 1784 bp to 2463 bp (590 aa to 782 aa and 3'-UTR),
Prey-No. 7: 1298 bp to 1991 bp (428 aa to 657 aa), and
Prey-No. 11: 1983 bp to 2618 bp (656 aa to 782 aa and 3'-UTR).
Full length 894 amino acid residues of Skeletal muscle alpha 2 actinin (Accession No. M86406)
Prey-No. 10: 1096 bp to 1755 bp (309 aa to 528 aa).

The myosin binding subunit 85 *alias* protein phosphatase I, regulatory (inhibitor) subunit 12C was reported as a novel gene in the June, 2001 issue of "The Journal of Biological Chemistry" (vol. 276, No. 24, 21209-21216). The N-terminus ankyrin repeat thereof (100 aa to 287 aa) binds to protein phosphatase 1δ (PP1δ), being the essential region for the actin depolymerization. In its middle region was found the MERC-alpha kinase phosphorylated domain that is phosphorylated by the MERC-α kinase. The myosin binding subunit 85 has been known to cause the structural alteration by phosphorylation of this domain. As the other motifs of the myosin binding subunit 85 were found, in its C-terminus, the alpha-helical leucine repeat and phosphorylation inhibitory motif, the latter motif being the region having the inhibitory action against the ankyrin repeat.

The skeletal muscle alpha 2 actinin is a molecule already well known as the actin-binding regulatory protein. Since both of these two genes are associated with the actin depolymerization, the involvement of B1153 gene in the actin depolymerization was suggested. A possibility was suggested that, in T-cells, the B1153 gene acts in the reconstruction of cytoskeleton, and is involved in the activation, cell division, and migration of T-cells.

Therefore, a compound that suppresses the interaction of B1153 protein with the myosin binding subunit 85 or the skeletal muscle alpha 2 actinin is expected to have therapeutic effects on an allergic disease based on the inhibition of the B1153 protein activity. Activity of test compound on the interaction of these proteins can be easily valued with the binding reaction between proteins as an index. For example, it is also possible to carry out the high throughput screening for assessing the interference of a test compound using the immobilized B1153 protein, and labeled myosin binding subunit 85 or skeletal muscle alpha 2 actinin. That is, the B1153 protein of this invention enables a high throughput screening for detecting a compound useful in the treatment of an allergic disease.

### Industrial Applicability

The present invention provided a gene that shows the difference in its expression levels between normal healthy subjects and allergic disease patients. Using the expression of the gene of this invention as an index, it became possible to test for an allergic disease, and screen for a candidate compound for a therapeutic agent for the disease. In particular, by using the activity of B1153 protein that was revealed in this invention as an index, a high throughput screening system can be easily constructed.

Expression levels of allergic disease-associated genes provided by the present invention can be easily detected regardless of the types of allergens. Therefore, pathological conditions of allergic diseases can be comprehensively understood.

In addition, using peripheral blood leukocytes as a specimen, the expression level of genes can be analyzed in a much less invasive manner to patients according to the method for testing for allergic disease of the present invention. Furthermore, according to the gene expression analysis method of the present invention, in contrast to protein measurements such as ECP, highly sensitive measurement with a trace sample can be accomplished. Gene analysis technique trends toward high-throughput and lower prices. Therefore, the test method according to the present invention is expected to become an important bedside diagnostic method in the near future. In this sense, these genes associated with pathological conditions are highly valuable in diagnosis.

Furthermore, the screening methods of the present invention are performed using, as an index, the genes whose expression are commonly observed among allergic disease patients. Therefore, compounds that can be detected according to these screening methods are expected to be useful in controlling a wide range of allergic pathological conditions.

## Claims

1. A method of testing for an allergic disease, said method comprising the following steps of:
a) measuring the expression level of a gene having the nucleotide sequence of SEQ ID NO: 1 in T-cells of a subject; and
b) comparing the expression level of the gene with that in T-cells of a normal healthy subject.

2. The testing method according to claim 1, wherein the allergic disease is atopic dermatitis.

3. The testing method according to claim 1, wherein the gene expression level is measured by PCR of the cDNA of the gene.

4. The testing method according to claim 1, wherein the gene expression level is measured by detecting a protein encoded by the gene.

5. A reagent for testing for an allergic disease, said reagent comprising an oligonucleotide that has a nucleotide sequence complementary to the polynucleotide sequence of SEQ ID NO: 1 or to a complementary strand thereof and that is at least 15 nucleotides long.

6. A reagent for testing for an allergic disease, said reagent comprising an antibody that recognizes a peptide comprising the amino acid sequence of SEQ ID NO: 2.

7. A method of detecting the effect of a candidate compound on the expression level of a polynucleotide according to any one of the following (a) through (d) , said method comprising the following steps of:
(1) contacting a candidate compound with a cell that expresses a polynucleotide of any one of the following (a) through (d),
(a) a polynucleotide comprising the coding region of the nucleotide sequence of SEQ ID NO: 1,
(b) a polynucleotide encoding a protein comprising the amino acid sequence of SEQ ID NO: 2,
(c) a polynucleotide encoding a protein comprising the amino acid sequence of SEQ ID NO: 2 in which one or more amino acids are substituted, deleted, inserted and/or added and which is functionally equivalent to the protein whose expression level is increased in T-cells of an allergic disease patient, and
(d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient; and
(2) measuring the expression level of the polynucleotide according to any one of the above-described (a) through (d).

8. The method according to claim 7, wherein said cell is a T cell line.

9. A method of detecting the effect of a candidate compound on the expression level of a polynucleotide according to any one of the following (a) through (d):
(a) a polynucleotide comprising the coding region of the nucleotide sequence of SEQ ID NO: 1,
(b) a polynucleotide encoding a protein comprising the amino acid sequence of SEQ ID NO: 2,
(c) a polynucleotide encoding a protein comprising the amino acid sequence of SEQ ID NO: 2 in which one or more amino acids are substituted, deleted, inserted and/or added and that is functionally equivalent to the protein whose expression level is increased in T-cells of an allergic disease patient, and
(d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient,
wherein said method comprises the following steps of:
(1) administering a candidate compound to a test animal, and
(2) measuring the expression level of the polynucleotide according to any one of the above-described (a) through (d) in T-cells of the test animal.

10. A method of screening for a compound that reduces the expression level of a polynucleotide according to any one of the above-described (a) through (d), said method comprising the steps of detecting the effect of a candidate compound on said expression level by the method according to claim 7 or 9, and selecting a compound that reduces said expression level compared to a control.

11. A method of detecting the effect of a candidate compound on the activity of a transcriptional regulatory region of a gene having the nucleotide sequence of SEQ ID NO: 1, said method comprising the following steps of:
(1) contacting a candidate compound with a cell into which a vector has been introduced, wherein the vector contains the transcriptional regulatory region of the gene having the nucleotide sequence of SEQ ID NO: 1 and a reporter gene that is expressed under the control of said transcriptional regulatory region, and
(2) measuring the activity of said reporter gene.

12. A method of screening for a compound that reduces the activity of the transcriptional regulatory region of the gene having the nucleotide sequence of SEQ ID NO: 1, said method comprising the steps of detecting the effect of a candidate compound on said activity by the method according to claim 11, and selecting a compound that reduces said activity compared to a control.

13. A method of detecting the effect of a candidate compound on the activity of a protein encoded by a polynucleotide according to any one of (a) through (d), said method comprising the following steps of:
(1) contacting a candidate compound with a protein encoded by a polynucleotide according to any one of the following (a) through (d),
(a) a polynucleotide comprising the coding region of the nucleotide sequence of SEQ ID NO: 1,
(b) a polynucleotide encoding a protein comprising the amino acid sequence of SEQ ID NO: 2,
(c) a polynucleotide encoding a protein comprising the amino acid sequence of SEQ ID NO: 2 in which one or more amino acids are substituted, deleted, inserted and/or added and that is functionally equivalent to the protein whose expression level is increased in T-cells of an allergic disease patient, and
(d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient; and
(2) measuring the activity of said protein.

14. The method according to claim 13, wherein said activity of a protein is its binding activity to myosin binding subunit 85 or skeletal muscle alpha 2 actinin.

15. A method of screening for a compound that reduces the activity of a protein encoded by a polynucleotide according to any one of (a) through (d) , said method comprising the steps of detecting the effect of a candidate compound on said activity by the method according to claim 13, and selecting a compound that reduces said activity compared to a control.

16. A vector containing a transcriptional regulatory region of a gene having the nucleotide sequence of SEQ ID NO: 1 and a reporter gene that is expressed under the control of said transcriptional regulatory region.

17. A cell into which the vector according to claim 16 has been introduced.

18. A therapeutic agent for an allergic disease, said agent comprising a compound obtainable by the screening method according to any one of claims 10, 12 and 15 as an effective ingredient.

19. A therapeutic agent for an allergic disease, said agent comprising, as a principal ingredient, an antisense DNA against a polynucleotide having the nucleotide sequence of SEQ ID NO: 1, or a portion thereof.

20. A therapeutic agent for an allergic disease, said agent comprising, as a principal ingredient, an antibody that binds to a protein having the amino acid sequence of SEQ ID NO: 2.

21. A polynucleotide according to any one of the following (a) through (d):
(a) a polynucleotide comprising the coding region of the nucleotide sequence of SEQ ID NO: 1,
(b) a polynucleotide encoding a protein comprising the amino acid sequence of SEQ ID NO: 2,
(c) a polynucleotide encoding a protein comprising the amino acid sequence of SEQ ID NO: 2 in which one or more amino acids are substituted, deleted, inserted, and/or added that is functionally equivalent to the protein whose expression level is increased in T-cells of an allergic disease patient, and
(d) a polynucleotide hybridizing to a DNA comprising a nucleotide sequence selected from that of SEQ ID NO: 1 under stringent conditions, wherein the polynucleotide encodes a protein whose expression level is increased in T-cells of an allergic disease patient.

22. A protein encoded by the polynucleotide according to claim 21.

23. A vector harboring the polynucleotide according to claim 21 in an expressible manner.

24. A transformed cell harboring the polynucleotide according to claim 21 or the vector according to claim 23.

25. A method of preparing the protein according to claim 22, said method comprising the steps of culturing the transformed cells according to claim 24, and collecting an expression product thereof.

26. An antibody against the protein according to claim 22.

27. A method of immunologically measuring the protein according to claim 22, said method comprising the step of observing the immunoreaction of the antibody according to claim 26 with the protein according to claim 22.

28. An oligonucleotide that has a nucleotide sequence complementary to the polynucleotide sequence of SEQ ID NO: 1 or to a complementary strand thereof and that is at least 15 nucleotides long.

29. A method of measuring the polynucleotide according to claim 21, wherein said method comprising the step of observing hybridization of the oligonucleotide according to claim 28 to the polynucleotide according to claim 21.

30. An allergic disease animal model comprising a transgenic non-human vertebrate in which the expression level of a polynucleotide according to any one of the following (a) through (d) is elevated in its T-cells:
(a) a polynucleotide comprising the coding region of the nucleotide sequence of SEQ ID NO: 1,
(b) a polynucleotide encoding a protein comprising the amino acid sequence of SEQ ID NO: 2,
(c) a polynucleotide encoding a protein comprising the amino acid sequence ser forth in SEQ ID NO: 2 in which one or more amino acids are substituted, deleted, inserted, and/or added and that is functionally equivalent to the protein whose expression level is increased in T-cells of an allergic disease patient, and
(d) a polynucleotide hybridizing to a DNA comprising a nucleotide sequence of SEQ ID NO: 1 under stringent conditions,
wherein the polynucleotide encodes a protein whose expression level is increased in T-cells of an allergic disease patient.

31. A kit for screening for a candidate compound for a therapeutic agent for an allergic disease, said kit comprising a polynucleotide that hybridizes to the nucleotide sequence of SEQ ID NO: 1 or to a complementary sequence thereof and that is at least 15 nucleotides long, and a cell expressing the gene comprising the nucleotide sequence of SEQ ID NO: 1.

32. A kit for screening for a candidate compound for a therapeutic agent for an allergic disease, said kit comprising an antibody that recognizes a peptide comprising an amino acid sequence selected from that of SEQ ID NO: 2, and a cell expressing the gene comprising the nucleotide sequence of SEQ ID NO: 1.

33. A kit for screening for a candidate compound for a therapeutic agent for an allergic disease, said kit comprising a protein comprising the amino acid sequence of SEQ ID NO: 2 and a protein that interacts with said protein.

34. The kit according to claim 33, wherein the interacting protein is a protein selected from the group consisting of myosin binding subunit 85, skeletal muscle alpha 2 actinin, and a fragment comprising an interacting domain thereof.
